Europäisches Patentamt
European Patent Office
Office européen des brevets

(19)

(11) Veröffentlichungsnummer: **0 560 330 A2**

(12) **EUROPÄISCHE PATENTANMELDUNG**

(21) Anmeldenummer: **93103854.1**

(22) Anmeldetag: **10.03.93**

(51) Int. Cl.[5]: **C07D 235/18**, C07D 235/08, C07D 471/04, C07D 403/14, A61K 31/415, //(C07D471/04, 235:00,221:00)

(30) Priorität: **12.03.92 DE 4207904**

(43) Veröffentlichungstag der Anmeldung: **15.09.93 Patentblatt 93/37**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach(DE)**

(72) Erfinder: **Hauel, Norbert, Dr.**
**Marderweg 12**
**W-7957 Schemmerhofen(DE)**
Erfinder: **Ries, Uwe, Dr.**
**Dunantstrasse 10**
**W-7950 Biberach 1(DE)**
Erfinder: **Narr, Berthold, Dr.**
**Obere Au 5**
**W-7950 Biberach 1(DE)**
Erfinder: **van Meel, Jacques, Dr.**
**Schubertweg 4**
**W-7951 Mittelbiberach(DE)**
Erfinder: **Wienen, Wolfgang, Dr.**
**Am Schiessberg 28**
**W-7951 Äpfingen(DE)**
Erfinder: **Entzeroth, Michael, Dr.**
**Sebastian-Sailer-Strasse 44**
**W-7951 Warthausen(DE)**

(54) **Substituierte Benzimidazolylderivate, diese Verbindungen enthaltende Arzneimittel und Verfahren zu ihrer Herstellung.**

(57) Die Erfindung betrifft substituierte Benzimidazolylderivate der allgemeinen Formel

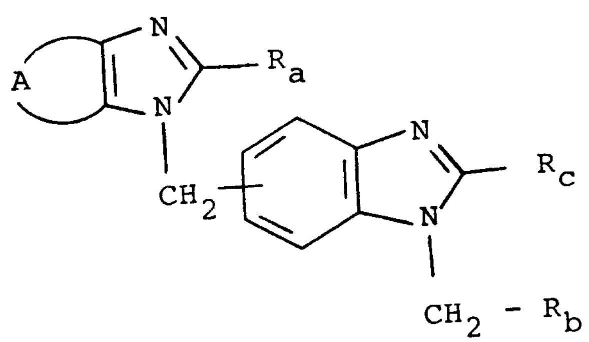

,(I)

in der
A und $R_a$ bis $R_c$ wie im Anspruch 1 definiert sind, deren Gemische von Stellungsisomerenmeren und deren Salze, welche wertvolle Eigenschaften aufweisen.

Diese weisen wertvolle pharmakologische Eigenschaften auf, insbesondere Angiotensin-antagonistische Wirkungen, vorzugsweise Angiotensin-II-antagonistische Wirkungen.

EP 0 560 330 A2

Die vorliegende Erfindung betrifft neue substituierte Benzimidazolylderivate der allgemeinen Formel

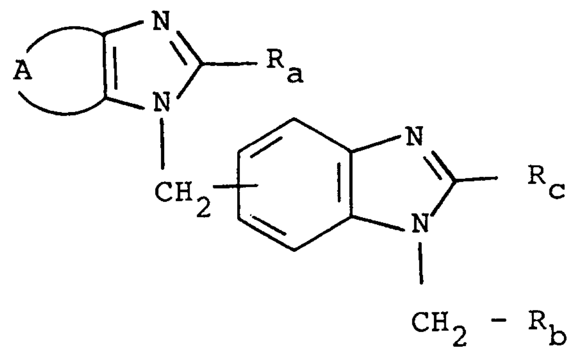

,(I)

deren Gemische von Stellungsisomeren sowie deren Salze, insbesondere für die pharmazeutische Anwendung deren physiologisch verträgliche Salze mit anorganischen oder organischen Säuren oder Basen, welche wertvolle pharmakologische Eigenschaften aufweisen, insbesondere Angiotensin-antagonistische Wirkungen, vorzugsweise Angiotensin-II-antagonistische Wirkungen.

In der obigen allgemeinen Formel bedeuten

A eine 1,4-Butadienylengruppe, die durch die Reste $R_1$ und $R_2$ substituiert ist und in der zusätzlich eine unsubstituierte Methingruppe durch ein Stickstoffatom ersetzt sein kann, wobei

$R_1$ ein Wasserstoff-, Fluor-, Chlor- oder Bromatom, eine Trifluormethylgruppe oder eine Alkylgruppe mit 1 bis 3 Kohlenstoffatomen und

$R_2$ ein Wasserstoffatom, eine Alkylgruppe mit 1 bis 3 Kohlenstoffatomen,

eine Carboxygruppe, eine Alkoxycarbonylgruppe mit insgesamt 2 bis 6 Kohlenstoffatomen,

eine Alkoxygruppe mit 2 bis 5 Kohlenstoffatomen, die in 2-, 3-, 4- oder 5-Position durch eine Imidazolyl-, Benzimidazolyl- oder Tetrahydrobenzimidazolylgruppe

substituiert ist,

eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können,

eine Phthalimino- oder Homophthaliminogruppe, wobei eine Carbonylgruppe in einer Phthaliminogruppe durch eine Methylengruppe ersetzt sein kann,

eine 5-, 6- oder 7-gliedrige Alkyleniminogruppe, in welcher eine Methylengruppe durch eine Carbonyl- oder Sulfonylgruppe ersetzt sein kann,

eine Glutarsäureiminogruppe, in der die n-Propylengruppe durch ein oder zwei Alkylgruppen mit jeweils 1 bis 3 Kohlenstoffatomen oder durch eine Tetramethylen- oder Pentamethylengruppe substituiert sein kann,

eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,

eine gegebenenfalls in 1-Stellung durch eine Alkylgruppe mit 1 bis 6 Kohlenstoffatomen oder eine Cycloalkylgruppe mit 3 bis 7 Kohlenstoffatomen substituierte Benzimidazol-2-yl-gruppe, wobei der Phenylkern in einer vorstehend erwähnten Benzimidazol-2-ylgruppe zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-, Imidazo[1,2-a]pyrimidin-2-yl-, Imidazo[4,5-b]pyridin-2-yl-, Imidazo[4,5-c]pyridin-2-yl-, Imidazo[1,2-c]pyrimidin-2-yl-, Imidazo[1,2-a]pyrazin-2-yl-, Imidazo[1,2-b]-pyridazin-2-yl-, Imidazo[4,5-c]pyridin-2-yl-, Purin-8-yl-, Imidazo[4,5-b]pyrazin-2-yl-, Imidazo[4,5-c]pyridazin-2-yl- oder Imidazo[4,5-d]pyridazin-2-yl-gruppe,

einen über ein Kohlenstoffatom gebundener Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonyl- oder Sulfonylgruppe ersetzt sein kann,

eine gegebenenfalls in 2-Stellung durch eine Alkylgruppe mit 1 bis 6 Kohlenstoffatomen oder durch eine Phenylgruppe substituierte Imidazol-4-yl-Gruppe, die in 1-Stellung durch eine Alkylgruppe mit 1 bis 7 Kohlenstoffatomen, die in 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxidothiomorpholinocarbonylgruppe substituiert sein kann, durch eine Alkylgruppe mit 2 bis 4

Kohlenstoffatomen, die in 2-, 3- oder 4-Stellung durch eine Hydroxy-, Alkoxy- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine gegebenenfalls durch Fluor- oder Chloratome, durch Trifluormethyl-, Methyl- oder Methoxygruppen mono- oder disubstituierte Phenylgruppe substituiert ist, durch eine durch zwei Phenylgruppen substituierte Alkylgruppe oder durch eine Cycloalkylgruppe mit 3 bis 7 Kohlenstoffatomen substituiert ist, wobei, soweit nichts anderes erwähnt wurde, die vorstehend erwähnten Alkyl- und Alkoxyteile jeweils 1 bis 3 Kohlenstoffatome enthalten können,
eine Carboxy-, Aminocarbonyl-, Alkylaminocarbonyl- oder Dialkylaminogruppe, in denen der Alkylteil jeweils 1 bis 6 Kohlenstoffatome enthalten kann, oder eine Gruppe, die in-vivo metabolisch in eine Carboxygruppe umgewandelt wird, oder
eine $R_5$-$NR_4$-CO-$NR_3$-Gruppe, in der

$R_3$ ein Wasserstoffatom, eine Alkylgruppe mit 1 bis 5 Kohlenstoffatomen, eine Cyclohexyl- oder Benzylgruppe,

$R_4$ ein Wasserstoffatom, eine Alkylgruppe mit 1 bis 6 Kohlenstoffatomen, eine Allyl-, Cyclohexyl-, Benzyl- oder Phenylgruppe,

$R_5$ ein Wasserstoffatom oder eine Alkylgruppe mit 1 bis 3 Kohlenstoffatomen oder

$R_4$ und $R_5$ zusammen mit dem dazwischen liegenden Stickstoffatom eine unverzweigte Cycloalkyleniminogruppe mit 4 bis 6 Kohlenstoffatomen oder eine Morpholinogruppe oder

$R_3$ und $R_4$ zusammen eine Alkylengruppe mit 2 oder 3 Kohlenstoffatomen darstellen,

$R_a$ eine Alkylgruppe mit 1 bis 5 Kohlenstoffatomen, eine Cycloalkylgruppe mit 3 bis 5 Kohlenstoffatomen, eine Alkoxy-, Alkylthio- oder Alkylaminogruppe mit jeweils 1 bis 3 Kohlenstoffatomen in jedem Alkylteil,

$R_b$ eine Carboxy-, Cyano-, Hydroxysulfonyl-, 1H-Tetrazolyl-, 1-Triphenylmethyl-tetrazolyl- oder 2-Triphenylmethyl-tetrazolylgruppe, eine Gruppe, die in-vivo metabolisch in eine Carboxygruppe umgewandelt wird, eine Alkancarbonylaminosulfonyl-, Benzoylaminosulfonyl-, Alkansulfonylaminocarbonyl-, Trifluormethansulfonylaminocarbonyl- oder Phenylsulfonylaminocarbonylgruppe, wobei in den vorstehend erwähnten Gruppen die Alkyl- und Alkoxyteile jeweils 1 bis 4 Kohlenstoffatome enthalten können, und

$R_c$ eine Alkylgruppe, eine Cycloalkylgruppe mit 3 bis 6 Kohlenstoffatomen oder eine Phenylgruppe, die durch ein Fluor-, Chlor- oder Bromatom, durch eine Hydroxy-, Alkoxy- oder Alkylgruppe mono- oder disubstituiert sein kann, wobei die Substituenten gleich oder verschieden sein können und die in den vorstehend erwähnten Gruppen erwähnten Alkyl- und Alkoxyteile jeweils 1 bis 6 Kohlenstoffatome enthalten können.

Unter dem vorstehend erwähnten Begriff "eine Gruppe, die in-vivo metabolisch in eine Carboxygruppe umgewandelt wird," sind beispielsweise deren Ester der Formeln

- CO - OR',
- CO - O - (HCR'') - O - CO - R''' und
- CO - O - (HCR'') - O - CO - OR'''

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten, zu verstehen.

Die neuen Verbindungen der obigen Formel I weisen wertvolle Eigenschaften auf. So weisen die Verbindungen der Formel I, in der $R_b$ eine Gruppe, die in-vivo metabolisch in eine Carboxygruppe umgewandelt wird, eine Carboxy- oder 1H-Tetrazolylgruppe bedeutet, insbesondere wertvolle pharmakologische Eigenschaften auf, da diese Angiotensin-Antagonisten, insbesondere Angiotensin-II-Antagonisten, darstellen. Die übrigen Verbindungen der allgemeinen Formel I, in der $R_b$ beispielsweise die Cyano-, 1-Triphenylmethyl-tetrazolyl- oder 2-Triphenylmethyl-tetrazolylgruppe darstellt, stellen wertvolle Zwischenprodukte zur Herstellung der vorstehend erwähnten Verbindungen dar.

Gegenstand der vorliegenden Erfindung sind somit die neuen Benzimidazol-1-yl-, Imidazo[4,5-b]pyridin-1-yl-, Imidazo[4,5-c]pyridin-1-yl-, Imidazo[4,5-b]pyridin-3-yl- und Imidazo[4,5-c]pyridin-3-yl-benzimidazolylmethyl-Derivate der obigen allgemeinen Formel I, deren Gemische von Stellungsisomeren und deren Salze, insbesondere für die pharmazeutische Anwendung, deren verträglichen Salze, und Verfahren zu ihrer Herstellung.

Ein weiterer Gegenstand der vorliegenden Erfindung sind somit neue Arzneimittel, welche eine der oben erwähnten pharmakologisch wirksamen Verbindungen der allgemeinen Formel I oder ein entsprechendes physiologisch verträgliches Salz enthalten und insbesondere zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen, Blasenerkrankungen, zur Prävention atherosklerotischer Gefäßveränderungen und zur Prävention von Restenosen nach operativer Erweiterung von Blutgefäß-Stenosen.

Für die bei der Definition der Reste $R_a$ bis $R_b$ eingangs erwähnten Bedeutungen kommt beispielsweise Für $R_a$ die Bedeutung der Methyl-, Ethyl-, n-Propyl-, Isopropyl-, n-Butyl-, 1-Methyl-n-propyl-, 2-Methyl-n-propyl-, tert.Butyl-, n-Pentyl-, 1-Methyl-1-butyl-, 2-Methyl-1-butyl-, 3-Methyl-1-butyl-, 1,1-Dimethyl-1-propyl-, 2,2-Dimethyl-1-propyl-, Cyclopropyl-, Cyclobutyl-, Cyclopentyl-, Methoxy-, Ethoxy-, n-Propoxy-, Isopropoxy-, Methylthio-, Ethylthio-, n-Propylthio-, Isopropylthio-, Methylamino-, Ethylamino-, n-Propylamino- oder Isopropylaminogruppe,

für $R_b$ die Hydroxycarbonyl-, Methoxycarbonyl-, Ethoxycarbonyl-, n-Propyloxycarbonyl-, Isopropyloxycarbonyl-, n-Butyloxycarbonyl-, Isobutyloxycarbonyl-, tert.Butyloxycarbonyl-, n-Pentyloxycarbonyl-, Isoamyloxycarbonyl-, n-Hexyloxycarbonyl-, Cyclopentyloxycarbonyl-, Cyclohexyloxycarbonyl-, Benzyloxycarbonyl-, 1-Phenylethyloxycarbonyl-, 2-Phenylethyloxycarbonyl-, 3-Phenylpropyloxycarbonyl-, Methoxymethoxycarbonyl-, Cinnamyloxycarbonyl-, Acetoxymethoxycarbonyl-, Propionyloxymethoxycarbonyl-, n-Butyryloxymethoxycarbonyl-, Isobutyryloxymethoxycarbonyl-, n-Pentanoyloxymethoxycarbonyl-, Isopentanoyloxymethoxycarbonyl-, Pivaloyloxymethoxycarbonyl-, n-Hexanoyloxymethoxycarbonyl-, Cyclopentanoyloxymethoxycarbonyl-, Cyclohexanoyloxymethoxycarbonyl-, Phenylacetoxymethoxycarbonyl-, 2-Phenylpropionyloxymethoxycarbonyl-, 3-Phenylpropionyloxymethoxycarbonyl-, 4-Phenylbutyryloxymethoxycarbonyl-, Benzoyloxymethoxycarbonyl-, 1-Acetoxyethoxycarbonyl-, 1-Propionyloxyethoxycarbonyl-, 1-n-Butyryloxyethoxycarbonyl-, 1-Isobutyryloxyethoxycarbonyl-, 1-n-Pentanoyloxyethoxycarbonyl-, 1-Isopentanoyloxyethoxycarbonyl-, 1-Pivaloyloxyethoxycarbonyl-, 1-n-Hexanoyloxyethoxycarbonyl-, 1-Cyclopentanoyloxyethoxycarbonyl-, 1-Cyclohexanoyloxyethoxycarbonyl-, 1-Phenylacetoxyethoxycarbonyl-, 1-(2-Phenylpropionyloxy)-ethoxycarbonyl-, 1-(3-Phenylpropionyloxy)-ethoxycarbonyl-, 1-(4-Phenylbutyryloxy)-ethoxycarbonyl-, 1-Benzoyloxyethoxycarbonyl-, Methoxycarbonyloxymethoxycarbonyl-, Ethoxycarbonyloxymethoxycarbonyl-, n-Propyloxycarbonyloxymethoxycarbonyl-, Isopropyloxycarbonyloxymethoxycarbonyl-, n-Butyloxycarbonyloxymethoxycarbonyl-,Isobutyloxycarbonyloxymethoxycarbonyl-, tert.Butyloxycarbonyloxymethoxycarbonyl-, n-Pentyloxycarbonyloxymethoxycarbonyl-,Isoamyloxycarbonyloxymethoxycarbonyl-, n-Hexyloxycarbonyloxymethoxycarbonyl-, Cyclopentyloxycarbonyloxymethoxycarbonyl-,Cyclohexyloxycarbonyloxymethoxycarbonyl-, Benzyloxycarbonyloxymethoxycarbonyl-, 1-Phenylethoxycarbonyloxymethoxycarbonyl-, 2-Phenylethoxycarbonyloxymethoxycarbonyl-, 3-Phenylpropyloxycarbonyloxymethoxycarbonyl-, Cinnamyloxycarbonyloxymethoxycarbonyl-, 1-(Methoxycarbonyloxy)-ethoxycarbonyl-, 1-(Ethoxycarbonyloxy)-ethoxycarbonyl-, 1-(n-Propyloxycarbonyloxy)-ethoxycarbonyl-, 1-(Isopropyloxycarbonyloxy)-ethoxycarbonyl-, 1-(n-Butyloxycarbonyloxy)-ethoxycarbonyl-, 1-(Isobutyloxycarbonyloxy)-ethoxycarbonyl-, 1-(tert.Butyloxycarbonyloxy)-ethoxycarbonyl-, 1-(n-Pentyloxycarbonyloxy)-ethoxycarbonyl-, 1-(Isoamyloxycarbonyloxy)-ethoxycarbonyl-, 1-(n-Hexyloxycarbonyloxy)-ethoxycarbonyl-, 1-(Cyclopentyloxycarbonyloxy)-ethoxycarbonyl-, 1-(Cyclohexyloxycarbonyloxy)-ethoxycarbonyl-, 1-(Benzyloxycarbonyloxy)-ethoxycarbonyl-, 1-(1-Phenylethoxycarbonyloxy)-ethoxycarbonyl-, 1-(2-Phenylethoxycarbonyloxy)-ethoxycarbonyl-, 1-(3-Phenylpropyloxycarbonyloxy)-ethoxycarbonyl-, 1-(Cinnamyloxycarbonyloxy)-ethoxycarbonyl-, Hydroxysulfonyl-, Cyano-, 1H-Tetrazolyl-, 1-Triphenylmethyl-tetrazolyl-, 2-Triphenylmethyl-tetrazolyl-, Trifluormethansulfonylaminocarbonyl-, Methansulfonylaminocarbonyl-, Ethansulfonylaminocarbonyl-, n-Propansulfonylaminocarbonyl-, Isopropansulfonylaminocarbonyl-, Phenylsulfonylaminocarbonyl-, 4-Fluorphenylsulfonylaminocarbonyl-, 4-Chlorphenylsulfonylaminocarbonyl-, 4-Bromphenylsulfonylaminocarbonyl-, 4-Methylphenylsulfonylaminocarbonyl-, 4-Methoxyphenylsulfonylaminocarbonyl-, Methylcarbonylaminosulfonyl-, Ethylcarbonylaminosulfonyl-, n-Propylcarbonylaminosulfonyl-, n-Butylcarbonylaminosulfonyl- oder Benzoylaminosulfonylgruppe,

für $R_c$ die der Methyl-, Ethyl-, n-Propyl-, Isopropyl-, Isobutyl-, n-Butyl-, 1-Methyl-n-propyl-, 2-Methyl-n-propyl-, tert.Butyl-, n-Pentyl-, 1-Methyl-n-butyl-, 2-Methyl-n-butyl-, n-Hexyl-, 1-Methyl-n-pentyl-, 2-Methyl-n-pentyl-, 3-Methyl-n-pentyl-, Cyclopropyl-, Cyclobutyl-, Cyclopentyl-, Cyclohexyl-, Phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 2-Methyl-phenyl-, 3-Methyl-phenyl-, 4-Methyl-phenyl-, 2-Ethyl-phenyl-, 3-Ethyl-phenyl-, 4-Ethyl-phenyl-, 2-n-Propyl-phenyl-, 3-n-Butyl-phenyl-, 4-n-Pentyl-phenyl-, 2-Hydroxy-phenyl-, 3-Hydroxy-phenyl-, 4-Hydroxy-phenyl-, 2-Methoxy-phenyl-, 3-Methoxy-phenyl-, 4-Methoxy-phenyl-, 2-Ethoxy-phenyl-, 3-Ethoxy-phenyl-, 4-Ethoxy-phenyl-, 2-n-Propoxy-phenyl-, 3-n-Butoxy-phenyl-, 4-n-Hexoxyphenyl-, 2,4-Difluor-phenyl-, 2,5-Dichlor-phenyl-, 3,4-Dibrom-phenyl-, 2,4-Dimethyl-phenyl-, 3,4-Dimethoxyphenyl-, 3,4-Dihydroxy-phenyl- oder 2-Chlor-5-methoxy-phenylgruppe,

für $R_1$ die des Wasserstoff-, Fluor-, Chlor- oder Bromatoms, der Methyl-, Ethyl-, n-Propyl-, Isopropyl- oder Trifluormethylgruppe,

für $R_2$ die des Wasserstoffatoms, der Acetylamino-, Propionylamino-, Butanoylamino-, Pentanoylamino-, Benzoylamino-, N-Acetyl-methylamino-, N-Propionyl-methylamino-, N-Butanoyl-methylamino-, N-Pentanoylmethylamino-, N-Benzoyl-methylamino-, N-Acetyl-ethylamino-, N-Propionyl-ethylamino-, N-Butanoyl-ethylamino-,N-Pentanoyl-ethylamino-, N-Benzoyl-ethylamino-, N-Acetyl-isopropylamino-, N-Propionyl-n-propylamino-, N-Butanoyl-n-propylamino-, N-Pentanoyl-isopropylamino-, N-Benzoyl-isopropylamino-, 2-(Imidazol-1-yl)-ethoxy-, 3-(Imidazol-1-yl)-propoxy-, 4-(Imidazol-1-yl)-butoxy-, 5-(Imidazol-1-yl)-pentoxy-, 2-(Benzimidazol-1-yl)-ethoxy-, 3-(Benzimidazol-1-yl)-propoxy-, 4-(Benzimidazol-1-yl)-butoxy-, 5-(Benzimidazol-1-yl)-pentoxy-, 2-(Tetrahydrobenzimidazol-1-yl)-ethoxy-, 3-(Tetrahydrobenzimidazol-1-yl)-propoxy-, 4-(Tetrahydrobenzimidazol-1-yl)-butoxy-, 5-(Tetrahydrobenzimidazol-1-yl)-pentoxy-, Carboxy-, Methoxycarbonyl-, Ethoxycarbonyl-, Isopropoxycarbonyl-, Pentoxycarbonyl-, Phthalimino-, Homophthalimino-, 1-Oxo-isoindolin-2-yl-, Pyrrolidino-, Piperidino-, Hexamethylenimino-, 2-Oxo-pyrrolidino-, 2-Oxo-piperidino-, 2-Oxo-hexamethylenimino-, Propansultam-1-yl-, Butansultam-1-yl-, Pentansultam-1-yl-, Glutarimino-, 3,3-Tetramethylen-glutarimino-, 3,3-Pentamethylen-glutarimino-, 2,2-Dimethyl-glutarimino-, 3-Methyl-glutarimino-, 3,3-Dimethyl-glutarimino-, 3-Ethyl-glutarimino-, 3-Ethyl-3-methyl-glutarimino-, 1,3-Cyclopentandicarbonylimino-, 2,4-Dimethylglutarimino-, 2,4-Di-n-propyl-glutarimino-, Maleinsäureimido-, 2-Methyl-maleinsäureimido-, 2-Phenyl-maleinsäureimido-, 2,3-Dimethyl-maleinsäureimido-, 3-Methyl-2-phenyl-maleinsäureimido-,2,3-Diphenyl-maleinsäureamido-, Pyrrolidin-2-yl-, Pyrrolidin-2-on-5-yl-, Piperidin-2-yl-, Piperidin-2-on-1-yl-,Piperidin-2-on-6-yl-, Pyridin-2-yl-, Chinolin-2-yl-, Isochinolin-1-yl-, Isochinolin-3-yl-, 1-Methyl-imidazol-4-yl-, 1-Ethyl-imidazol-4-yl-, 1-n-Propyl-imidazol-4-yl-, 1-Isopropyl-imidazol-4-yl-, 1-n-Butyl-imidazol-4-yl-, 1-Isobutyl-imidazol-4-yl-, 1-n-Pentyl-imidazol-4-yl-, 1-Isoamyl-imidazol-4-yl-, 1-n-Hexyl-imidazol-4-yl-, 1-n-Hexyl-2-methyl-imidazol-4-yl-, 1-(1-Methyl-n-pentyl)-imidazol-4-yl-, 1-(1-Ethyl-n-butyl)-imidazol-4-yl-, 1-(1-Methyl-n-hexyl)-imidazol-4-yl-, 1-(1-Ethyl-n-pentyl)-imidazol-4-yl-, 1-(1-n-Propyl-n-butyl)-imidazol-4-yl-, 1-n-Heptyl-imidazol-4-yl-, 1-Ethyl-2-methyl-imidazol-4-yl-,1-n-Propyl-2-methyl-imidazol-4-yl-, 1-Isopropyl-2-methyl-imidazol-4-yl-, 1-n-Butyl-2-methyl-imidazol-4-yl-, 1-Isobutyl-2-methyl-imidazol-4-yl-, 1-n-Pentyl-2-methyl-imidazol-4-yl-, 1-Isoamyl-2-methyl-imidazol-4-yl-, 1-n-Hexyl-2-methyl-imidazol-4-yl-, 1-n-Heptyl-2-methyl-imidazol-4-yl-, 1-Cyclopropylmethyl-imidazol-4-yl-, 1-Cyclobutylmethyl-imidazol-4-yl-, 1-Cyclopentylmethyl-imidazol-4-yl-, 1-Cyclohexylmethyl-imidazol-4-yl-, 1-Cycloheptylmethyl-imidazol-4-yl-, 1-(2-Cyclopropylethyl)-imidazol-4-yl-, 1-(2-Cyclobutylethyl)-imidazol-4-yl-, 1-(2-Cyclopentylethyl)-imidazol-4-yl-, 1-(2-Cyclohexylethyl)-imidazol-4-yl-, 1-(2-Cycloheptylethyl)-imidazol-4-yl-, 1-(3-Cyclopropylpropyl)-imidazol-4-yl-, 1-(3-Cyclobutylpropyl)-imidazol-4-yl-, 1-(3-Cyclopentylpropyl)-imidazol-4-yl-, 1-(3-Cyclohexylpropyl)-imidazol-4-yl-, 1-(3-Cycloheptylpropyl)-imidazol-4-yl-, 1-(2,2,2-Trifluorethyl)-imidazol-4-yl-, 1-(3,3,3-Trifluorpropyl)-imidazol-4-yl-, 1-Benzyl-imidazol-4-yl-,1-(2-Phenylethyl)-imidazol-4-yl-, 1-(3-Phenylpropyl)-imidazol-4-yl-, 1-(4-Fluor-benzyl)-imidazol-4-yl-, 1-(4-Chlor-benzyl)-imidazol-4-yl-, 1-(3-Chlor-benzyl)-imidazol-4-yl-, 1-(4-Trifluormethyl-benzyl)-imidazol-4-yl-, 1-(3-Methyl-benzyl)-imidazol-4-yl-, 1-(4-Methyl-benzyl)-imidazol-4-yl-, 1-(3-Methoxy-benzyl)-imidazol-4-yl-, 1-(4-Methoxy-benzyl)-imidazol-4-yl-, 1-(3,4-Dimethoxy-benzyl)-imidazol-4-yl-, 1-(3,5-Dimethoxy-benzyl)-imidazol-4-yl-, 1-Cyclopropylmethyl-2-methyl-imidazol-4-yl-, 1-Cyclobutylmethyl-2-methyl-imidazol-4-yl-, 1-Cyclopentylmethyl-2-methyl-imidazol-4-yl-, 1-Cyclohexylmethyl-2-methyl-imidazol-4-yl-, 1-Cycloheptylmethyl-2-methyl-imidazol-4-yl-, 1-(2-Cyclopropylethyl)-2-methyl-imidazol-4-yl-, 1-(2-Cyclobutylethyl)-2-methyl-imidazol-4-yl-, 1-(2-Cyclopentylethyl)-2-methyl-imidazol-4-yl-, 1-(2-Cyclohexylethyl)-2-methyl-imidazol-4-yl-, 1-(2-Cycloheptylethyl)-2-methyl-imidazol-4-yl-, 1-(3-Cyclopropylpropyl)-2-methyl-imidazol-4-yl-, 1-(3-Cyclobutylpropyl)-2-methyl-imidazol-4-yl-, 1-(3-Cyclopentylpropyl)-2-methyl-imidazol-4-yl-, 1-(3-Cyclohexylpropyl)-2-methyl-imidazol-4-yl-, 1-(3-Cycloheptylpropyl)-2-methyl-imidazol-4-yl-, 1-(2,2,2-Trifluorethyl)-2-methyl-imidazol-4-yl-, 1-(3,3,3-Trifluorpropyl)-2-methyl-imidazol-4-yl-, 1-Benzyl-2-methyl-imidazol-4-yl-, 1-(2-Phenylethyl)-2-methyl-imidazol-4-yl-, 1-(3-Phenylpropyl)-2-methyl-imidazol-4-yl-, 1-(4-Fluor-benzyl)-2-methyl-imidazol-4-yl-, 1-(4-Chlor-benzyl)-2-methyl-imidazol-4-yl-, 1-(3-Chlor-benzyl)-2-methyl-imidazol-4-yl-, 1-(4-Trifluormethyl-benzyl)-2-methyl-imidazol-4-yl-, 1-(3-Methyl-benzyl)-2-methyl-imidazol-4-yl-, 1-(4-Methyl-benzyl)-2-methyl-imidazol-4-yl-, 1-(3-Methoxy-benzyl)-2-methyl-imidazol-4-yl-, 1-(4-Methoxy-benzyl)-2-methyl-imidazol-4-yl-, 1-(3,4-Dimethoxy-benzyl)-2-methyl-imidazol-4-yl-, 1-(3,5-Dimethoxy-benzyl)-2-methyl-imidazol-4-yl-, 1-Carboxymethyl-imidazol-4-yl-, 1-(2-Carboxyethyl)-imidazol-4-yl-, 1-(3-Carboxypropyl)-imidazol-4-yl-, 1-(4-Carboxybutyl)-imidazol-4-yl-, 1-(5-Carboxypentyl)-imidazol-4-yl-, 1-(6-Carboxyhexyl)-imidazol-4-yl-, 1-(7-Carboxyheptyl)-imidazol-4-yl-, 1-Methoxycarbonylmethyl-imidazol-4-yl-, 1-(2-Methoxycarbonylethyl)-imidazol-4-yl-, 1-(3-Methoxycarbonylpropyl)-imidazol-4-yl-, 1-(4-Methoxycarbonylbutyl)-imidazol-4-yl-, 1-(5-Methoxycarbonylpentyl)-imidazol-4-yl-, 1-(6-Methoxycarbonylhexyl)-imidazol-4-yl-, 1-(7-Methoxycarbonylheptyl)-imidazol-4-yl-, 1-Ethoxycarbonylmethyl-imidazol-4-yl-, 1-(2-Ethoxycarbonylethyl)-imidazol-4-yl-, 1-(3-Ethoxycarbonylpropyl)-imidazol-4-yl-, 1-(4-Ethoxycarbonylbutyl)-imidazol-4-yl-, 1-(5-Ethoxycarbonylpentyl)-imidazol-4-yl-, 1-(6-Ethoxycarbonylhexyl)-imidazol-4-yl-, 1-(7-Ethoxycarbonylheptyl)-imidazol-4-yl-, 1-n-Propoxycarbonylmethyl-imidazol-4-yl-, 1-(2-n-Propoxycarbonylethyl)-imidazol-4-yl-, 1-(3-n-Propoxycarbonylpropyl)-imidazol-4-yl-, 1-(4-n-Propoxycarbonylbutyl)-imidazol-4-yl-, 1-(5-n-Propoxycarbonylpentyl)-imidazol-4-yl-, 1-(6-n-Propoxycarbonylhexyl)-imidazol-4-yl-, 1-(7-n-Propoxycarbonylheptyl)-imidazol-4-yl-, 1-Isopropoxycarbonylmethyl-imidazol-4-yl-, 1-(2-Isopropoxycarbonylethyl)-imidazol-4-yl-, 1-(3-Isopropoxycarbonylpropyl)-imidazol-4-yl-, 1-(4-Isopropoxycarbonylbutyl)-imidazol-4-yl-, 1-(5-Isopropoxycarbonylpentyl)-imidazol-4-yl-, 1-(6-Isopropoxycarbonylhexyl)-imidazol-4-yl-, 1-(7-Isopropoxycarbonylheptyl)-imidazol-4-yl-, 1-Aminocarbonylmethyl-imidazol-4-yl-, 1-(2-Aminocarbonylethyl)-imidazol-4-yl-, 1-(3-Aminocarbonylpropyl)-imidazol-4-yl-, 1-(4-Aminocarbonylbutyl)-imidazol-4-yl-, 1-(5-Aminocarbonylpentyl)-imidazol-4-yl-, 1-(6-Aminocarbonylhexyl)-imidazol-4-yl-, 1-(7-Aminocarbonylheptyl)-imidazol-4-yl-, 1-Methylaminocarbonylmethyl-imidazol-4-yl-, 1-(2-Methylaminocarbonylethyl)-imidazol-4-yl-, 1-(3-Methylaminocarbonylpropyl)-imidazol-4-yl-, 1-(4-Methylaminocarbonylbutyl)-imidazol-4-yl-, 1-(5-Methylaminocarbonylpentyl)-imidazol-4-yl-, 1-(6-Methylaminocarbonylhexyl)-imidazol-4-yl-, 1-(7-Methylaminocarbonylheptyl)-imidazol-4-yl-, 1-Ethylaminocarbonylmethyl-imidazol-4-yl-, 1-(2-Ethylaminocarbonylethyl)-imidazol-4-yl-, 1-(3-Ethylaminocarbonylpropyl)-imidazol-4-yl-, 1-(4-Ethylaminocarbonylbutyl)-imidazol-4-yl-, 1-(5-Ethylaminocarbonylpentyl)-imidazol-4-yl-, 1-(6-Ethylaminocarbonylhexyl)-imidazol-4-yl-, 1-(7-Ethylaminocarbonylheptyl)-imidazol-4-yl-, 1-n-Propylaminocarbonylmethyl-imidazol-4-yl-, 1-(2-n-Propylaminocarbonylethyl)-imidazol-4-yl-, 1-(3-n-Propylaminocarbonylpropyl)-imidazol-4-yl-, 1-(4-n-Propylaminocarbonylbutyl)-imidazol-4-yl-, 1-(5-n-Propylaminocarbonylpentyl)-imidazol-4-yl-, 1-(6-n-Propylaminocarbonylhexyl)-imidazol-4-yl-, 1-(7-n-Propylaminocarbonylheptyl)-imidazol-4-yl-, 1-Isopropylaminocarbonylmethyl-imidazol-4-yl-, 1-(2-Isopropylaminocarbonylethyl)-imidazol-4-yl-, 1-(3-Isopropylaminocarbonylpropyl)-imidazol-4-yl-, 1-(4-Isopropylaminocarbonylbutyl)-imidazol-4-yl-, 1-(5-Isopropylaminocarbonylpentyl)-imidazol-4-yl-, 1-(6-Isopropylaminocarbonylhexyl)-imidazol-4-yl-, 1-(7-Isopropylaminocarbonylheptyl)-imidazol-4-yl-, 1-Dimethylaminocarbonylmethyl-imidazol-4-yl-, 1-(2-Dimethylaminocarbonylethyl)-imidazol-4-yl-, 1-(3-Dimethylaminocarbonylpropyl)-imidazol-4-yl-, 1-(4-Dimethylaminocarbonylbutyl)-imidazol-4-yl-, 1-(5-Dimethylaminocarbonylpentyl)-imidazol-4-yl-, 1-(6-Dimethylaminocarbonylhexyl)-imidazol-4-yl-, 1-(7-Dimethylaminocarbonylheptyl)-imidazol-4-yl-, 1-Diethylaminocarbonylmethyl-imidazol-4-yl-, 1-(2-Diethylaminocarbonylethyl)-imidazol-4-yl-, 1-(3-Diethylaminocarbonylpropyl)-imidazol-4-yl-, 1-(4-Diethylaminocarbonylbutyl)-imidazol-4-yl-, 1-(5-Diethylaminocarbonylpentyl)-imidazol-4-yl-, 1-(6-Diethylaminocarbonylhexyl)-imidazol-4-yl-, 1-(7-Diethylaminocarbonylheptyl)-imidazol-4-yl-, 1-Di-n-propylaminocarbonylmethyl-imidazol-4-yl-, 1-(2-Di-n-propylaminocarbonylethyl)-imidazol-4-yl-, 1-(3-Di-n-propylaminocarbonylpropyl)-imidazol-4-yl-, 1-(4-Di-n-propylaminocarbonylbutyl)-imidazol-4-yl-, 1-(5-Di-n-propylaminocarbonylpentyl)-imidazol-4-yl-, 1-(6-Di-n-propylaminocarbonylhexyl)-imidazol-4-yl-, 1-(7-Di-n-propylaminocarbonylheptyl)-imidazol-4-yl-, 1-Diisopropylaminocarbonylmethyl-imidazol-4-yl-, 1-(2-Diisopropylaminocarbonylethyl)-imidazol-4-yl-, 1-(3-Diisopropylaminocarbonylpropyl)-imidazol-4-yl-, 1-(4-Diisopropylaminocarbonylbutyl)-imidazol-4-yl-, 1-(5-Diisopropylaminocarbonylpentyl)-imidazol-4-yl-, 1-(6-Diisopropylaminocarbonylhexyl)-imidazol-4-yl-, 1-(7-Diisopropylaminocarbonylheptyl)-imidazol-4-yl-, 1-Morpholinocarbonylmethyl-imidazol-4-yl-, 1-(2-Morpholinocarbonylethyl)-imidazol-4-yl-, 1-(3-Morpholinocarbonylpropyl)-imidazol-4-yl-, 1-(4-Morpholinocarbonylbutyl)-imidazol-4-yl-, 1-(5-Morpholinocarbonylpentyl)-imidazol-4-yl-, 1-(6-Morpholinocarbonylhexyl)-imidazol-4-yl-, 1-(7-Morpholinocarbonylheptyl)-imidazol-4-yl-, 1-Thiomorpholinocarbonylmethyl-imidazol-4-yl-, 1-(2-Thiomorpholinocarbonylethyl)-imidazol-4-yl-, 1-(3-Thiomorpholinocarbonylpropyl)-imidazol-4-yl-, 1-(4-Thiomorpholinocarbonylbutyl)-imidazol-4-yl-, 1-(5-Thiomorpholinocarbonylpentyl)-imidazol-4-yl-, 1-(6-Thiomorpholinocarbonylhexyl)-imidazol-4-yl-, 1-(7-Thiomorpholinocarbonylheptyl)-imidazol-4-yl-, 1-Oxidothiomorpholinocarbonylmethyl-imidazol-4-yl-, 1-(2-Oxidothiomorpholinocarbonylethyl)-imidazol-4-yl-, 1-(3-Oxidothiomorpholinocarbonylpropyl)-imidazol-4-yl-, 1-(4-Oxidothiomorpholinocarbonylbutyl)-imidazol-4-yl-, 1-(5-Oxidothiomorpholinocarbonylpentyl)-imidazol-4-yl-, 1-(6-Oxidothiomorpholinocarbonylhexyl)-imidazol-4-yl-, 1-(7-Oxidothiomorpholinocarbonylheptyl)-imidazol-4-yl-, 1-Carboxymethyl-2-methyl-imidazol-4-yl-, 1-(2-Carboxyethyl)-2-methyl-imidazol-4-yl-, 1-(3-Carboxypropyl)-2-methyl-imidazol-4-yl-, 1-(4-Carboxybutyl)-2-methyl-imidazol-4-yl-, 1-(5-Carboxypentyl)-2-methyl-imidazol-4-yl-, 1-(6-Carboxyhexyl)-2-methyl-imidazol-4-yl-, 1-(7-Carboxyheptyl)-2-methyl-imidazol-4-yl-, 1-Methoxycarbonylmethyl-2-methyl-imidazol-4-yl-, 1-(2-Methoxycarbonylethyl)-2-methyl-imidazol-4-yl-, 1-(3-Methoxycarbonylpropyl)-2-methyl-imidazol-4-yl-, 1-(4-Methoxycarbonylbutyl)-2-methyl-imidazol-4-yl-, 1-(5-Methoxycarbonylpentyl)-2-methyl-imidazol-4-yl-, 1-(6-Methoxycarbonylhexyl)-2-methyl-imidazol-4-yl-, 1-(7-Methoxycarbonylheptyl)-2-methyl-imidazol-4-yl-, 1-Ethoxycarbonylmethyl-2-methyl-imidazol-4-yl-, 1-(2-Ethoxycarbonylethyl)-2-methyl-imidazol-4-yl-, 1-(3-Ethoxycarbonylpropyl)-2-methyl-imidazol-4-yl-, 1-(4-Ethoxycarbonylbutyl)-2-methyl-imidazol-4-yl-, 1-(5-Ethoxycarbonylpentyl)-2-methyl-imidazol-4-yl-, 1-(6-Ethoxycarbonylhexyl)-2-methyl-imidazol-4-yl-, 1-(7-Ethoxycarbonylheptyl)-2-methyl-imidazol-4-yl-, 1-n-Propoxycarbonylmethyl-2-methyl-imidazol-4-yl-, 1-(2-n-Propoxycarbonylethyl)-2-methyl-imidazol-4-yl-, 1-(3-n-Propoxycarbonylpropyl)-2-methyl-imidazol-4-yl-, 1-(4-n-Propoxycarbonylbutyl)-2-methyl-imidazol-4-yl-, 1-(5-n-Propoxycarbonylpentyl)-2-methyl-imidazol-4-yl-, 1-(6-n-Propoxycarbonylhexyl)-2-methyl-imidazol-4-yl-, 1-(7-n-Propoxycarbonylheptyl)-2-methyl-imidazol-4-yl-, 1-Isopropoxycarbonylmethyl-2-methyl-imidazol-4-yl-, 1-(2-Isopropoxycarbonylethyl)-2-methyl-imidazol-4-yl-, 1-(3-Isopropoxycarbonylpropyl)-2-methyl-imidazol-4-yl-, 1-(4-Isopropoxycarbonylbutyl)-2-methyl-imidazol-4-yl-, 1-(5-Isopropoxycarbonylpentyl)-2-methyl-imidazol-4-yl-, 1-(6-Isopropoxycarbonylhexyl)-2-methyl-imidazol-4-yl-, 1-(7-Isopropoxycarbonylheptyl)-2-methyl-imidazol-4-yl-, 1-Aminocarbonylmethyl-2-methyl-imidazol-4-yl-, 1-(2-Aminocarbonylethyl)-2-methyl-imidazol-4-yl-, 1-(3-Aminocarbonylpropyl)-2-methyl-imidazol-4-yl-, 1-(4-Aminocarbonylbutyl)-2-methyl-imidazol-4-yl-, 1-(5-Aminocarbonylpentyl)-2-methyl-imidazol-4-yl-, 1-(6-Aminocarbonylhexyl)-2-methyl-imidazol-4-yl-, 1-(7-Aminocarbonylheptyl)-2-methyl-imidazol-4-yl-, 1-Methylaminocarbonylmethyl-2-methyl-imidazol-4-yl-, 1-(2-Methylaminocarbonylethyl)-2-methyl-imidazol-4-yl-, 1-(3-Methylaminocarbonylpropyl)-2-methyl-imidazol-4-yl-, 1-(4-Methylaminocarbonylbutyl)-2-methyl-imidazol-4-yl-, 1-(5-Methylaminocarbonylpentyl)-2-methyl-imidazol-4-yl-, 1-(6-Methylaminocarbonylpentyl)-2-methyl-imidazol-4-yl-, 1-(7-Methylaminocarbonylheptyl)-2-methyl-imidazol-4-yl-, 1-Ethylaminocarbonylmethyl-2-methyl-imidazol-4-yl-, 1-(2-Ethylaminocarbonylethyl)-2-methyl-imidazol-4-yl-, 1-(3-Ethylaminocarbonylpropyl)-2-methyl-imidazol-4-yl-, 1-(4-Ethylaminocarbonylbutyl)-2-methyl-imidazol-4-yl-, 1-(5-Ethylaminocarbonylpentyl)-2-methyl-imidazol-4-yl-, 1-(6-Ethylaminocarbonylhexyl)-2-methyl-imidazol-4-yl-, 1-(7-Ethylaminocarbonylheptyl)-2-methyl-imidazol-4-yl-, 1-n-Propylaminocarbonylmethyl-2-methyl-imidazol-4-yl-, 1-(2-n-Propylaminocarbonylethyl)-2-methyl-imidazol-4-yl-, 1-(3-n-Propylaminocarbonylpropyl)-2-methyl-imidazol-4-yl-, 1-(4-n-Propylaminocarbonylbutyl)-2-methyl-imidazol-4-yl-, 1-(5-n-Propylaminocarbonylpentyl)-2-methyl-imidazol-4-yl-, 1-(6-n-Propylaminocarbonylhexyl)-2-methyl-imidazol-4-yl-, 1-(7-n-Propylaminocarbonylheptyl)-2-methyl-imidazol-4-yl-, 1-Isopropylaminocarbonylmethyl-2-methyl-imidazol-4-yl-, 1-(2-Isopropylaminocarbonylethyl)-2-methyl-imidazol-4-yl-, 1-(3-Isopropylaminocarbonylpropyl)-2-methyl-imidazol-4-yl-, 1-(4-Isopropylaminocarbonylbutyl)-2-methyl-imidazol-4-yl-, 1-(5-Isopropylaminocarbonylpentyl)-2-methyl-imidazol-4-yl-, 1-(6-Isopropylaminocarbonylhexyl)-2-methyl-imidazol-4-yl-, 1-(7-Isopropylaminocarbonylheptyl)-2-methyl-imidazol-4-yl-, 1-Dimethylaminocarbonylmethyl-2-methyl-imidazol-4-yl-, 1-(2-Dimethylaminocarbonylethyl)-2-methyl-imidazol-4-yl-, 1-(3-Dimethylaminocarbonylpropyl)-2-methyl-imidazol-4-yl-, 1-(4-Dimethylaminocarbonylbutyl)-2-methyl-imidazol-4-yl-, 1-(5-Dimethylaminocarbonylpentyl)-2-methyl-imidazol-4-yl-, 1-(6-Dimethylaminocarbonylhexyl)-2-methyl-imidazol-4-yl-, 1-(7-Dimethylaminocarbonylheptyl)-2-methyl-imidazol-4-yl-, 1-Diethylaminocarbonylmethyl-2-methyl-imidazol-4-yl-, 1-(2-Diethylaminocarbonylethyl)-2-methyl-imidazol-4-yl-, 1-(3-Diethylaminocarbonylpropyl)-2-methyl-imidazol-4-yl-, 1-(4-Diethylaminocarbonylbutyl)-2-methyl-imidazol-4-yl-, 1-(5-Diethylaminocarbonylpentyl)-2-methyl-imidazol-4-yl-, 1-(6-Diethylaminocarbonylhexyl)-2-methyl-imidazol-4-yl-, 1-(7-Diethylaminocarbonylheptyl)-2-methyl-imidazol-4-yl-, 1-Di-n-propylaminocarbonylmethyl-2-methyl-imidazol-4-yl-, 1-(2-Di-n-propylaminocarbonylethyl)-2-methyl-imidazol-4-yl-, 1-(3-Di-n-propylaminocarbobylpropyl)-2-methyl-imidazol-4-yl-, 1-(4-Di-n-propylaminocarbonylbutyl)-2-methyl-imidazol-4-yl-, 1-(5-Di-n-propylaminocarbonylpentyl)-2-methyl-imidazol-4-yl-, 1-(6-Di-n-propylaminocarbonylhexyl)-2-methyl-imidazol-4-yl-, 1-(7-Di-n-propylaminocarbobylheptyl)-2-methyl-imidazol-4-yl-, 1-Diisopropylaminocarbonylmethyl-2-methyl-imidazol-4-yl-, 1-(2-Diisopropylaminocarbonylethyl)-2-methyl-imidazol-4-yl-, 1-(3-Diisopropylaminocarbonylpropyl)-2-methyl-imidazol-4-yl-, 1-(4-Diisopropylaminocarbonylbutyl)-2-methyl-imidazol-4-yl-, 1-(5-Diisopropylaminocarbonylpentyl)-2-methyl-imidazol-4-yl-, 1-(6-Diisopropylaminocarbonylhexyl)-2-methyl-imidazol-4-yl-, 1-(7-Diisopropylaminocarbonylheptyl)-2-methyl-imidazol-4-yl-, 1-Morpholinocarbonylmethyl-2-methyl-imidazol-4-yl-, 1-(2-Morpholinocarbonylethyl)-2-methyl-imidazol-4-yl-, 1-(3-Morpholinocarbonylpropyl)-2-methyl-imidazol-4-yl-, 1-(4-Morpholinocarbonylbutyl)-2-methyl-imidazol-4-yl-, 1-(5-Morpholinocarbonylpentyl)-2-methyl-imidazol-4-yl-, 1-(6-Morpholinocarbonylhexyl)-2-methyl-imidazol-4-yl-, 1-(7-Morpholinocarbonylheptyl)-2-methyl-imidazol-4-yl-, 1-Thiomorpholinocarbonylmethyl-2-methyl-imidazol-4-yl-, 1-(2-Thiomorpholinocarbonylethyl)-2-methyl-imidazol-4-yl-, 1-(3-Thiomorpholinocarbonylpropyl)-2-methyl-imidazol-4-yl-, 1-(4-Thiomorpholinocarbonylbutyl)-2-methyl-imidazol-4-yl-, 1-(5-Thiomorpholinocarbonylpentyl)-2-methyl-imidazol-4-yl-, 1-(6-Thiomorpholinocarbonylhexyl)-2-methyl-imidazol-4-yl-, 1-(7-Tniomorpholinocarbonylheptyl)-2-methyl-imidazol-4-yl-, 1-Oxidothiomorpholinocarbonylmethyl-2-methyl-imidazol-4-yl-, 1-(2-Oxidothiomorpholinocarbonylethyl)-2-methyl-imidazol-4-yl-, 1-(3-Oxidothiomorpholinocarbonylpropyl)-2-methyl-imidazol-4-yl-, 1-(4-Oxidothiomorpholinocarbonylbutyl)-2-methyl-imidazol-4-yl-, 1-(5-Oxidothiomorpholinocarbonylpentyl)-2-methyl-imidazol-4-yl-, 1-(6-Oxidothiomorpholinocarbonylhexyl)-2-methyl-imidazol-4-yl-, 1-(7-Oxidothiomorpholinocarbonylheptyl)-2-methyl-imidazol-4-yl-, 1-(2-Hydroxyethyl)-imidazol-4-yl-, 1-(3-Hydroxypropyl)-imidazol-4-yl-, 1-(4-Hydroxybutyl)-imidazol-4-yl-, 1-(2-Methoxyethyl)-imidazol-4-yl-, 1-(3-Methoxypropyl)-imidazol-4-yl-, 1-(4-Methoxybutyl)-imidazol-4-yl-, 1-(2-Ethoxyethyl)-imidazol-4-yl-, 1-(3-Ethoxypropyl)-imidazol-4-yl-, 1-(4-Ethoxybutyl)-imidazol-4-yl-, 1-(2-n-Propoxyethyl)-imidazol-4-yl-, 1-(3-n-Propoxypropyl)-imidazol-4-yl-, 1-(4-n-Propoxybutyl)-imidazol-4-yl-, 1-(2-Isopropoxyethyl)-imidazol-4-yl-, 1-(3-Isopropoxypropyl)-imidazol-4-yl-, 1-(4-Isopropoxybutyl)-imidazol-4-yl-, 1-(2-Imidazol-1-yl-ethyl)-imidazol-4-yl-, 1-(3-Imidazol-1-yl-propyl)-imidazol-4-yl-, 1-(4-Imidazol-1-yl-butyl)-imidazol-4-yl-, 1-(2,2-Diphenyl-ethyl)-imidazol-4-yl-, 1-(3,3-Diphenyl-propyl)-imidazol-4-yl-, 1-(4,4-Diphenyl-butyl)-imidazol-4-yl-, 1-(2-Hydroxyethyl)-2-methyl-imidazol-4-yl-, 1-(3-Hydroxypropyl)-2-methyl-imidazol-4-yl-, 1-(4-Hydroxybutyl)-2-methyl-imidazol-4-yl-, 1-(2-Methoxyethyl)-2-methyl-imidazol-4-yl-, 1-(3-Methoxypropyl)-2-methyl-imidazol-4-yl-, 1-(4-Methoxybutyl)-2-methyl-imidazol-4-yl-, 1-(2-Ethoxyethyl)-2-methyl-imidazol-4-yl-, 1-(3-Ethoxypropyl)-2-methyl-imidazol-4-yl-, 1-(4-Ethoxybutyl)-2-methyl-imidazol-4-yl-, 1-(2-n-Propoxyethyl)-2-methyl-imidazol-4-yl-, 1-(3-n-Propoxypropyl)-2-methyl-imidazol-4-yl-, 1-(4-n-Propoxybutyl)-2-methyl-imidazol-4-yl-, 1-(2-Isopropoxyethyl)-2-methyl-imidazol-4-yl-, 1-(3-Isopropoxypropyl)-2-methyl-imidazol-4-yl-, 1-(4-Isopropoxybutyl)-2-methyl-imidazol-4-yl-, 1-(2-Imidazol-1-yl-ethyl)-2-methyl-imidazol-4-yl-, 1-(3-Imidazol-1-yl-propyl)-2-methyl-imidazol-4-yl-, 1-(4-Imidazol-1-yl-butyl)-2-methyl-imidazol-4-yl-, 1-(2,2-Diphenyl-ethyl)-2-methyl-imidazol-4-yl-, 1-(3,3-Diphenyl-propyl)-2-methyl-imidazol-4-yl-, 1-(4,4-Diphenyl-butyl)-2-methyl-imidazol-4-yl-, Benzimidazol-2-yl-, 1-Methylbenzimidazol-2-yl-, 1-Ethylbenzimidazol-2-yl-, 1-n-Propylbenzimidazol-2-yl-, 1-Isopropylbenzimidazol-2-yl-, 1-n-Butylbenzimidazol-2-yl-, 1-Isobutylbenzimidazol-2-yl-, 1-n-Pentylbenzimidazol-2-yl-, 1-n-Hexylbenzimidazol-2-yl-, 1-Cyclopropylbenzimidazol-2-yl-, 1-Cyclobutylbenzimidazol-2-yl-, 1-Cyclopentylbenzimidazol-2-yl-, 1-Cyclohexylbenzimidazol-2-yl-, 1,5-Dimethyl-benzimidazol-2-yl-, 1,6-Dimethyl-benzimidazol-2-yl-, 1,4-Dimethyl-benzimidazol-2-yl-,5-Fluor-1-methyl-benzimidazol-2-yl-, 6-Fluor-1-methyl-benzimidazol-2-yl-, 5-Trifluormethyl-benzimidazol-2-yl-, 5-Trifluormethyl-1-methyl-benzimidazol-2-yl-, Imidazo[1,2-a]pyridin-2-yl-, 5,6,7,8-Tetrahydro-imidazo[1,2-a]pyrimidin-2-yl-, Imidazo[1,2-a]pyrimidin-2-yl-, Imidazo[4,5-b]pyridin-2-yl-, Imidazo[4,5-c]pyridin-2-yl-, Imidazo[2,1-b]thiazol-6-yl, Imidazo[1,2-c]pyrimidin-2-yl-, Imidazo[1,2-a]pyrazin-2-yl-, Imidazo[1,2-b]pyidazin-2-yl-, Imidazo[4,5-c]pyridin-2-yl-, Purin-8-yl-, Imidazo[4,5-b]pyrazin-2-yl-, Imidazo[4,5-c]pyridazin-2-yl-, Imidazo[4,5-d]pyridazin-2-yl-,4,5-Dihydro-2H-pyridazin-3-on-6-yl-, 2-Methyl-4,5-dihydro-2H-pyridazin-3-on-6-yl-, 2-Benzyl-4,5-dihydro-2H-pyridazin-3-on-6-yl-, 2H-Pyridazin-3-on-6-yl-, 2-Methyl-pyridazin-3-on-6-yl-, 2-Benzyl-pyridazin-3-on-6-yl-, Aminocarbonylamino-, Methylaminocarbonylamino-, Dimethylaminocarbonylamino-, N-Methylaminocarbonyl-methylamino-, N-(Dimethylaminocarbonyl)-methylamino-,N-Dimethylaminocarbonyl-ethylamino-, N-Dimethylaminocarbonylisopropylamino-, N-(Dimethylaminocarbonyl)-n-pentylamino-, N-Methylaminocarbonyl-ethylamino-, N-Methylaminocarbonyl-n-pentylamino-, N-Methylaminocarbonyl-cyclohexylamino-, Ethylaminocarbonylamino-, N-Ethylaminocarbonyl-methylamino-, N-Ethylaminocarbonyl-ethylamino-, N-Ethylaminocarbonylcyclohexylamino-, Diethylaminocarbonylamino-, N-(Diethylaminocarbonyl)-methylamino-, N-(Diethylaminocarbonyl)-ethylamino-,N-(Diethylaminocarbonyl)-n-butylamino-, Isopropylaminocarbonylamino-, N-Isopropylaminocarbonyl-methylamino-, n-Butylaminocarbonylamino-, N-(n-Butylaminocarbonyl)-methylamino-, N-(n-Butylaminocarbonyl)-ethylamino-, N-(n-Butylaminocarbonyl)-isopropylamino-, N-(n-Butylaminocarbonyl)-n-butylamino-, N-(n-Butylaminocarbonyl)-cyclohexylamino-, N-(Di-(n-butyl)-aminocarbonyl)-amino-, N-(Di-(n-butyl)-aminocarbonyl)-methylamino-, N-(Di-(n-butyl)-aminocarbonyl)-ethylamino-, N-(Di-(n-butyl)-aminocarbonyl)-n-butylamino-, N-(n-Pentylaminocarbonyl)-methylamino-, N-(n-Pentylaminocarbonyl)-ethyl-amino-, N-(n-Hexylaminocarbonyl)-ethylamino-, n-Hexylaminocarbonylamino-, N-(n-Hexylaminocarbonyl)-n-butylamino-, N-(n-Hexylaminocarbonyl)-n-pentylamino-, N-(n-Hexylaminocarbonyl)-cyclohexylamino-, Di-(n-hexyl)-aminocarbonylamino-, N-(Di-(n-hexyl)-aminocarbonyl)-methylamino-, N-((n-hexyl)-methylaminocarbonyl)-amino-, Cyclohexylaminocarbonylamino-,N-Cyclohexylaminocarbonyl-methylamino-, N-Cyclohexylaminocarbonyl-ethylamino-, N-Cyclohexylaminocarbonyl-n-butylamino-, N-Cyclohexylaminocarbonyl-isobutylamino-, N-Cyclohexylaminocarbonyl-n-pentylamino-, N-Cyclohexylaminocarbonyl-cyclohexylamino-, N-(Ethyl-cyclohexylaminocarbonyl)-methylamino-, N-(Propyl-cyclohexylaminocarbonyl)-methylamino-, N-(n-Butyl-cyclohexylaminocarbonyl)-methylamino-, Allylaminocarbonylamino-,Benzylaminocarbonylamino-, N-Benzylaminocarbonyl-isobutylamino-, Phenylaminocarbonylamino-, Pyrrolidinocarbonylamino-, Pyrrolidinocarbonylmethylamino-, Piperidinocarbonylamino-, Hexamethyleniminocarbonylamino-, Morpholinocarbonylamino-, 3,4,5,6-Tetrahydro-2-pyrimidon-1-yl-, 3-Methyl-3,4,5,6-tetrahydro-2-pyrimidon-1-yl-, 3-Ethyl-3,4,5,6-tetrahydro-2-pyrimidon-1-yl-, 3-n-Propyl-3,4,5,6-tetrahydro-2-pyrimidon-1-yl-, 3-Isopropyl-3,4,5,6-tetrahydro-2-pyrimidon-1-yl-, Hydroxycarbonyl-, Methoxycarbonyl-, Ethoxycarbonyl-, n-Propyloxycarbonyl-, Isopropyloxycarbonyl-, n-Butyloxycarbonyl-, Isobutyloxycarbonyl-, tert.Butyloxycarbonyl-, n-Pentyloxycarbonyl-, Isoamyloxycarbonyl-, n-Hexyloxycarbonyl-, Cyclopentyloxycarbonyl-, Cyclohexyloxycarbonyl-, Benzyloxycarbonyl-, 1-Phenylethyloxycarbonyl-, 2-Phenylethyloxycarbonyl-, 3-Phenylpropyloxycarbonyl-, Methoxymethoxycarbonyl-, Cinnamyloxycarbonyl-, Acetoxymethoxycarbonyl-,

Propionyloxymethoxycarbonyl-, n-Butyryloxymethoxycarbonyl-, Isobutyryloxymethoxycarbonyl-, n-Pentanoyloxymethoxycarbonyl-, Isopentanoyloxymethoxycarbonyl-, Pivaloyloxymethoxycarbonyl-, n-Hexanoyloxymethoxycarbonyl-, Cyclopentanoyloxymethoxycarbonyl-, Cyclohexanoyloxymethoxycarbonyl-, Phenylacetoxymethoxycarbonyl-, 1-Phenylpropionyloxymethoxycarbonyl-, 2-Phenylpropionyloxymethoxycarbonyl-, 3-Phenylbutyryloxymethoxycarbonyl-, Benzoyloxymethoxycarbonyl-, 1-Acetoxyethoxycarbonyl-, 1-Propionyloxyethoxycarbonyl-, 1-n-Butyryloxyethoxycarbonyl-, 1-Isobutyryloxyethoxycarbonyl-, 1-n-Pentanoyloxyethoxycarbonyl-, 1-Isopentanoyloxyethoxycarbonyl-, 1-Pivaloyloxyethoxycarbonyl-, 1-n-Hexanoyloxyethoxycarbonyl-, 1-Cyclopentanoyloxyethoxycarbonyl-, 1-Cyclohexanoyloxyethoxycarbonyl-, 1-Phenylacetoxyethoxycarbonyl-, 1-(1-Phenylpropionyloxy)-ethoxycarbonyl-, 1-(2-Phenylpropionyloxy)-ethoxycarbonyl-, 1-(3-Phenylbutyryloxy)-ethoxycarbonyl-, 1-Benzoyloxyethoxycarbonyl-, Methoxycarbonyloxymethoxycarbonyl-, Ethoxycarbonyloxymethoxycarbonyl-, n-Propyloxycarbonyloxymethoxycarbonyl-, Isopropyloxycarbonyloxymethoxycarbonyl-, n-Butyloxycarbonyloxymethoxycarbonyl-, Isobutyloxycarbonyloxymethoxycarbonyl-, tert.Butyloxycarbonyloxymethoxycarbonyl-, n-Pentyloxycarbonyloxymethoxycarbonyl-, Isoamyloxycarbonyloxymethoxycarbonyl-, n-Hexyloxycarbonyloxymethoxycarbonyl-, Cyclopentyloxycarbonyloxymethoxycarbonyl-, Cyclohexyloxycarbonyloxymethoxycarbonyl-,Benzyloxycarbonyloxymethoxycarbonyl-, 1-Phenylethoxycarbonyloxymethoxycarbonyl-, 2-Phenylethoxycarbonyloxymethoxycarbonyl-, 3-Phenylpropyloxycarbonyloxymethoxycarbonyl-, Cinnamyloxycarbonyloxymethoxycarbonyl-, 1-(Methoxycarbonyloxy)-ethoxycarbonyl-, 1-(Ethoxycarbonyloxy)-ethoxycarbonyl-, 1-(n-Propyloxycarbonyloxy)-ethoxycarbonyl-, 1-(Isopropyloxycarbonyloxy)-ethoxycarbonyl-, 1-(n-Butyloxycarbonyloxy)-ethoxycarbonyl-, 1-(Isobutyloxycarbonyloxy)-ethoxycarbonyl-, 1-(tert.Butyloxycarbonyloxy)-ethoxycarbonyl-, 1-(n-Pentyloxycarbonyloxy)-ethoxycarbonyl-, 1-(Isoamyloxycarbonyloxy)-ethoxycarbonyl-, 1-(n-Hexyloxycarbonyloxy)-ethoxycarbonyl-, 1-(Cyclopentyloxycarbonyloxy)-ethoxycarbonyl-, 1-(Cyclohexyloxycarbonyloxy)-ethoxycarbonyl-, 1-(Benzyloxycarbonyloxy)-ethoxycarbonyl-, 1-(1-Phenylethoxycarbonyloxy)-ethoxycarbonyl -, 1-(2-Phenylethoxycarbonyloxy)-ethoxycarbonyl-, 1-(3-Phenylpropyloxycarbonyloxy)-ethoxycarbonyl-, 1-(Cinnamyloxycarbonyloxy)-ethoxycarbonyl-, Aminocarbonyl-, Methylaminocarbonyl-, Ethylaminocarbonyl-, n-Propylaminocarbonyl-, Isopropylaminocarbonyl-, Dimethylaminocarbonyl-, Diethylaminocarbonyl-, Di-n-propylaminocarbonyl-, Diisopropylaminocarbonyl-,N-Methyl-ethylaminocarbonyl- oder N-Ethyl-isopropylaminocarbonylgruppe in Betracht.

Bevorzugte Verbindungen der obigen allgemeinen Formel I sind diejenigen, in denen
A eine 1,4-Butadienylengruppe, die durch die Reste $R_1$ und $R_2$ substituiert ist und in der zusätzlich die Methingruppe in Position 7 des so gebildeten Benzimidazols durch ein Stickstoffatom ersetzt sein kann, wobei
$R_1$ ein Wasserstoffatom oder in 4-Stellung ein Fluor-, Chlor- oder Bromatom, eine Trifluormethylgruppe oder eine Alkylgruppe mit 1 bis 3 Kohlenstoffatomen und
$R_2$ ein Wasserstoffatom,
eine Alkylgruppe mit 1 bis 3 Kohlenstoffatomen,
in 6-Stellung eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können,
in 6-Stellung eine Phthalimino- oder Homophthaliminogruppe, wobei eine Carbonylgruppe in einer Phthaliminogruppe durch eine Methylengruppe ersetzt sein kann,
in 6-Stellung eine 5-, 6- oder 7-gliedrige Alkyleniminogruppe, in welcher eine Methylengruppe durch eine Carbonyl- oder Sulfonylgruppe ersetzt ist,
in 6-Stellung eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
in 6-Stellung eine gegebenenfalls in 1-Stellung durch eine Alkylgruppe mit 1 bis 6 Kohlenstoffatomen oder eine Cycloalkylgruppe mit 3 bis 7 Kohlenstoffatomen substituierte Benzimidazol-2-yl-gruppe, wobei der Phenylkern in einer vorstehend erwähnten Benzimidazol-2-ylgruppe zusätzlich durch ein Fluoratom, eine Methyl- oder Trifluormethylgruppe substituiert sein kann, eine Imidazo[2,1-b]thiazol-6-yl-, Imidazo[1,2-a]-pyridin-2-yl-, 5,6,7,8-Tetrahydro-imidazo[1,2-a]pyridin-2-yl-, Imidazo[1,2-a]pyrimidin-2-yl- oder Imidazo[4,5-b]pyridin-2-yl-gruppe,
in 6-Stellung eine Imidazol-4-yl-Gruppe, die in 1-Stellung durch eine Alkylgruppe mit 1 bis 7 Kohlenstoffatomen, die in 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxido-thiomorpholinocarbonylgruppe substituiert sein kann, durch eine Alkylgruppe mit 2 bis 4 Kohlenstoffatomen,

die in 2-, 3- oder 4-Stellung durch eine Hydroxy-, Alkoxy- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine gegebenenfalls durch Fluor- oder Chloratome, durch Trifluormethyl-, Methyl- oder Methoxygruppen mono- oder disubstituierte Phenylgruppe substituiert ist, durch eine durch zwei Phenylgruppen substituierte Alkylgruppe oder durch eine Cycloalkylgruppe mit 3 bis 7 Kohlenstoffatomen substituiert ist, wobei, soweit nichts anderes erwähnt wurde, die vorstehend erwähnten Alkyl- und Alkoxyteile jeweils 1 bis 3 Kohlenstoffatome enthalten können,

in 7-Stellung eine Carboxy-, Aminocarbonyl-, Alkylaminocarbonyl- oder Dialkylaminogruppe, in denen der Alkylteil jeweils 1 bis 6 Kohlenstoffatome enthalten kann, oder eine Gruppe, die in-vivo metabolisch in eine Carboxygruppe umgewandelt wird, oder

in 6-Stellung eine $R_5$-$NR_4$-CO-$NR_3$-Gruppe, in der

$R_3$ ein Wasserstoffatom, eine Alkylgruppe mit 1 bis 5 Kohlenstoffatomen, eine Cyclohexyl- oder Benzylgruppe,

$R_4$ ein Wasserstoffatom, eine Alkylgruppe mit 1 bis 6 Kohlenstoffatomen, eine Allyl-, Cyclohexyl-, Benzyl- oder Phenylgruppe,

$R_5$ ein Wasserstoffatom oder eine Alkylgruppe mit 1 bis 3 Kohlenstoffatomen oder

$R_4$ und $R_5$ zusammen mit dem dazwischen liegenden Stickstoffatom eine unverzweigte Cycloalkyleniminogruppe mit 4 bis 6 Kohlenstoffatomen oder eine Morpholinogruppe oder

$R_3$ und $R_4$ zusammen eine Alkylengruppe mit 2 oder 3 Kohlenstoffatomen darstellen,

$R_a$ eine Alkylgruppe mit 2 bis 4 Kohlenstoffatomen, eine Cycloalkylgruppe mit 3 oder 4 Kohlenstoffatomen oder eine Alkoxygruppe mit 2 oder 3 Kohlenstoffatomen,

$R_b$ eine Gruppe, die in-vivo metabolisch in eine Carboxygruppe umgewandelt wird, eine Carboxy- oder 1H-Tetrazolylgruppe und

$R_c$ eine Alkylgruppe mit 1 bis 3 Kohlenstoffatomen, eine Cycloalkylgruppe mit 3 bis 6 Kohlenstoffatomen oder eine Phenylgruppe bedeuten,

deren Gemische von Stellungsisomeren und deren Salze.

Besonders bevorzugte Verbindungen der obigen allgemeinen Formel I sind diejenigen, in denen

A eine 1,4-Butadienylengruppe, in der die Methingruppe in den Positionen 4 und 6 des so gebildeten Benzimidazols durch Methylgruppen substituiert sind und die Methingruppe in Position 7 durch ein Stickstoffatom ersetzt ist, oder

A eine 1,4-Butadienylengruppe, die durch die Reste $R_1$ und $R_2$ substituiert ist, wobei

$R_1$ ein Wasserstoffatom oder in 4-Stellung eine Methylgruppe und

$R_2$ in 6-Stellung eine 1-Methyl-benzimidazol-2-yl-Gruppe darstellen,

$R_a$ eine n-Alkylgruppe mit 2 bis 4 Kohlenstoffatomen,

$R_b$ eine Carboxy- oder 1H-Tetrazolylgruppe und

$R_c$ eine Alkylgruppe mit 1 bis 3 Kohlenstoffatomen, eine Cycloalkylgruppe mit 3 bis 6 Kohlenstoffatomen oder eine Phenylgruppe bedeuten,

deren Gemische von Stellungsisomeren und deren Salze.

Erfindungsgemäß erhält man die neuen Verbindungen nach folgenden Verfahren:

a) Umsetzung eines Benzimidazols der allgemeinen Formel

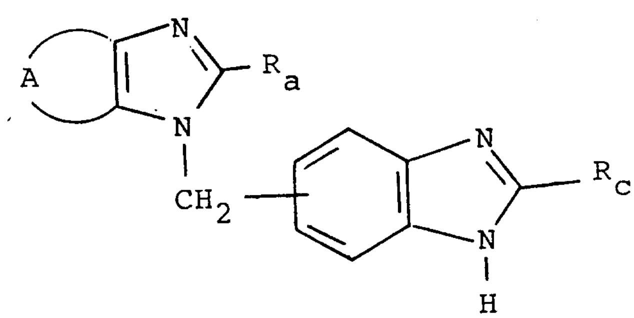

,(II)

in der

$R_a$, $R_c$ und A wie eingangs definiert sind, mit einer Verbindung der allgemeinen Formel

$Z_1$ - $CH_2$ - $R_b$ ,(III)

in der

$R_b$ mit Ausnahme der 1H-Tetrazolylgruppe wie eingangs definiert ist und
$Z_1$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy-oder p-Toluolsulfonyloxygruppe, darstellt, und gegebenenfalls anschließende Abspaltung einer 1-Triphenylmethylgruppe von einer so erhaltenen Triphenylmethyl-tetrazolylverbindung oder Hydrolyse eines so erhaltenen Esters.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Natriumhydrid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100 °C, z.B. bei Temperaturen zwischen Raumtemperatur und 50 °C, durchgeführt.

Die anschließende Abspaltung einer Triphenylmethylgruppe erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100 °C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150 °C, vorzugsweise bei Temperaturen zwischen 120 und 140 °C.

Die anschließende Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Bei der Umsetzung erhält man vorzugsweise ein Gemisch der 1- und 3-Isomeren, welches gewünschtenfalls anschließend, vorzugsweise chromatographisch unter Verwendung eines Trägers wie Kieselgel oder Aluminiumoxid, in das entsprechende 1- und 3-Isomere aufgetrennt wird.

b) Zur Herstellung einer Verbindung der allgemeinen Formel I, in der $R_b$ eine Carboxygruppe darstellt:
Überführung einer Verbindung der allgemeinen Formel

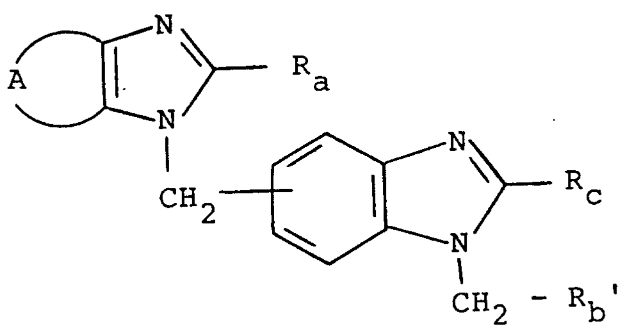

,(IV)

in der
$R_a$, $R_c$ und A wie eingangs definiert sind und
$R_b'$ eine mittels Hydrolyse, Thermolyse oder Hydrogenolyse in eine Carboxygruppe überführbare Gruppe darstellt, in eine entsprechende Carboxyverbindung.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_b$' in einer Verbindung der allgemeinen Formel IV eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50 ° C in die Carboxygruppe übergeführt werden.

Bedeutet $R_b$' in einer Verbindung der allgemeinen Formel IV beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 ° C und 100 ° C, abgespalten werden.

Bedeutet $R_b$' in einer Verbindung der allgemeinen Formel IV beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50 ° C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

c) Zur Herstellung einer Verbindung der allgemeinen Formel I, in der $R_b$ eine 1H-Tetrazolylgruppe darstellt:

Abspaltung eines Schutzrestes von einer Verbindung der allgemeinen Formel

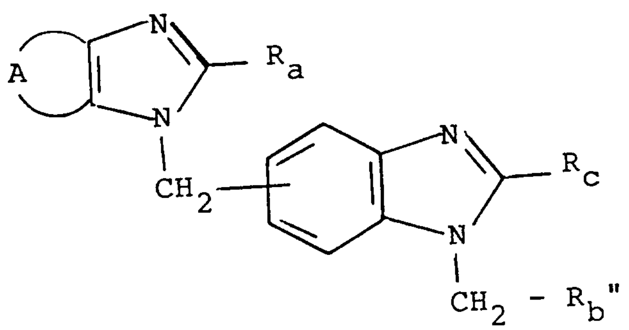

,(V)

in der

$R_a$, $R_c$ und A wie eingangs definiert sind und

$R_b$'' eine in 1- oder 2-Stellung durch einen Schutzrest geschützte 1H-Tetrazolylgruppe darstellt.

Als Schutzrest kommt beispielsweise die β-Cyanoethyl-, Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100 ° C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150 ° C, vorzugsweise bei Temperaturen zwischen 120 und 140 ° C.

d) Zur Herstellung einer Verbindung der allgemeinen Formel I, in der $R_b$ eine 1H-Tetrazolylgruppe darstellt:

Umsetzung einer Verbindung der allgemeinen Formel

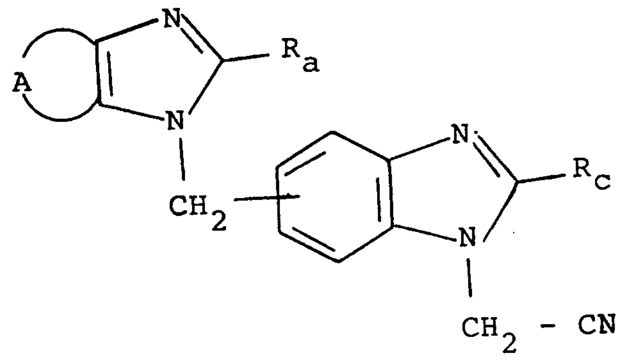

,(VI)

in der

$R_a$, $R_c$ und A wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150 °C, vorzugsweise bei 125 °C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

e) Zur Herstellung einer Verbindung der allgemeinen Formel I, in der $R_2$ eine $R_5$-$NR_4$-$CONR_3$-Gruppe darstellt:

Umsetzung einer Verbindung der allgemeinen Formel

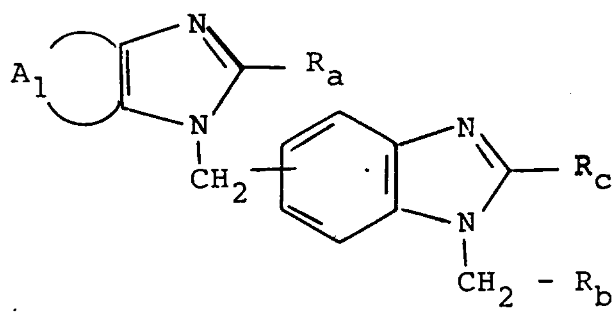

,(VII)

mit einer Verbindung der allgemeinen Formel

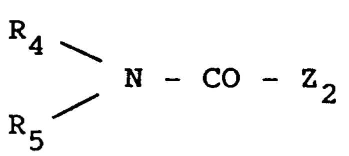

,(VIII)

in denen

$R_a$ bis $R_c$, $R_4$ und $R_5$ wie eingangs definiert sind,

$A_1$ eine 1,4-Butadienylengruppe, die durch $R_1$ und durch eine $R_3$NH-Gruppe substituiert ist, wobei $R_1$ und $R_3$ wie eingangs definiert sind, und

$Z_2$ eine nukleophile Austrittsgruppe wie ein Chlor- oder Bromatom oder auch

$Z_2$ und $R_5$ zusammen eine Stickstoff-Kohlenstoff-Bindung darstellen.

Die Umsetzung wird zweckmäßigerweise in einem Lösungsmittel oder Lösungsmittelgemisch wie Dichlormethan, Chloroform, Diethylether, Tetrahydrofuran, Dioxan, Dimethylformamid, Dimethylsulfoxid,

Pyridin, Benzol oder Toluol bei Temperaturen zwischen 0 und 150 °C, vorzugsweise jedoch bei Temperaturen zwischen 50 und 120 °C durchgeführt.

f) Zur Herstellung einer Verbindung der allgemeinen Formel I, in der $R_2$ eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können, eine Phthalimino- oder Homophthaliminogruppe, wobei eine Carbonylgruppe in einer Phthaliminogruppe durch eine Methylengruppe ersetzt sein kann, eine 5-, 6- oder 7-gliedrige Alkyleniminogruppe, in welcher eine Methylengruppe durch eine Carbonyl- oder Sulfonylgruppe ersetzt ist, eine Glutarsäureiminogruppe, in der die n-Propylengruppe durch ein oder zwei Alkylgruppen mit jeweils 1 bis 3 Kohlenstoffatomen oder durch eine Tetramethylen- oder Pentamethylengruppe substituiert sein kann, oder eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können, darstellt:

Umsetzung einer Verbindung der allgemeinen Formel

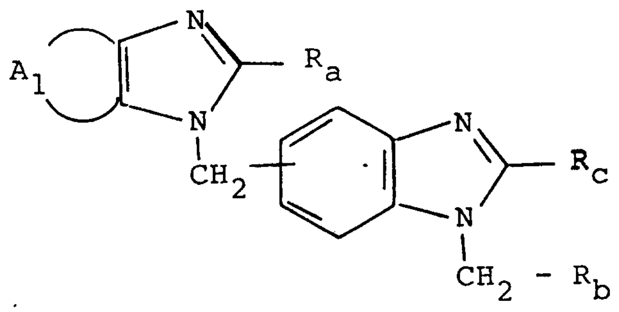

,(VII)

mit einer Verbindung der allgemeinen Formel

$Z_3$ - U - $R_6$ ,(IX)

in denen

$R_a$ bis $R_c$ wie eingangs definiert sind,

$A_1$ eine 1,4-Butadienylengruppe, die durch $R_1$ und durch eine $R_3$NH-Gruppe substituiert ist, wobei $R_1$ und $R_3$ wie eingangs definiert sind,

$Z_3$ eine Hydroxygruppe oder eine nukleophile Austrittsgruppe wie ein Halogenatom, z. B. ein Chlor- oder Bromatom,

U eine Carbonyl- oder Sulfonylgruppe und

$R_6$ eine Alkylgruppe mit 1 bis 4 Kohlenstoffatomen, eine Phenyl-, o-Hydroxycarbonylphenyl-, o-Hydroxycarbonylphenylmethyl-oder o-Hydroxycarbonylmethylphenylgruppe, eine gegebenenfalls durch eine oder zwei Alkylgruppen mit jeweils 1 bis 3 Kohlenstoffatomen oder durch eine Tetramethylen- oder Pentamethylengruppe substituierte 3-Hydroxycarbonylpropylgruppe oder eine gegebenenfalls durch eine Alkylgruppe mit 1 bis 3 Kohlenstoffatomen oder durch eine Phenylgruppe mono- oder disbustituierte 2-Hydroxycarbonylethenylgruppe, in der die Substituenten gleich oder verschieden sein können, oder

$R_3$ und $R_6$ zusammen eine n-Propylen-, n-Butylen- oder n-Pentylengruppe bedeuten, oder, falls $Z_3$ eine Hydroxygruppe darstellt, mit deren reaktionsfähigen Derivaten wie deren Säurehalogeniden, Säureanhydriden oder Säureestern.

Als reaktionsfähige Derivate einer Verbindung der Formel IX kommen beispielsweise deren Ester wie der Methyl-, Ethyl- oder Benzylester, deren Thioester wie der Methylthio- oder Ethylthioester, deren Halogenide wie das Säurechlorid, deren Anhydride oder Imidazolide und deren Orthoester in Betracht.

Die Umsetzung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid oder in einem Überschuß des Acylierungsmittels als Lösungsmittel mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und

100 °C, vorzugsweise jedoch bei Temperaturen zwischen -10 und 80 °C, durchgeführt.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Ein so erhaltenes Isomerengemisch einer Verbindung der allgemeinen Formel I kann gewünschtenfalls vorzugsweise chromatographisch unter Verwendung eines Trägers wie Kieselgel oder Aluminiumoxid getrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis IX sind teilweise literaturbekannt oder man erhält diese nach literaturbekannten Verfahren.

So erhält man beispielsweise eine Verbindung der allgemeinen Formel II durch Acylierung einer entsprechenden o-Amino-nitroverbindung, anschließende Reduktion der Nitrogruppe und anschließende Cyclisierung.

Die als Ausgangsstoffe verwendeten Verbindungen der allgemeinen Formeln IV, V, VI und VII erhält man durch Cyclisierung eines entsprechenden o-Phenylendiamins oder durch Reduktion einer entsprechenden o-Amino-nitroverbindung, anschließender Reduktion der Nitrogruppe, Cyclisierung einer so erhaltenen o-Diaminophenylverbindung und durch NH-Alkylierung eines so erhaltenen entsprechenden 1H-Benzimidazols, wobei das so erhaltene Isomerengemisch anschließend mittels üblicher Methoden, z.B. mittels Chromatographie, aufgetrennt werden kann.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 1-Hydroxycarbonylmethyl-2-phenyl-5-[(2-ethyl-4,6-dimethyl-imidazo[4,5-b]pyridin-1-yl)-methyl]-benzimidazoltrihydrat,

B = 1-Hydroxycarbonylmethyl-2-phenyl-6-[(2-n-propyl-4-methyl-6-(1-methyl-benzimidazol-2-yl)-benzimidazol-1-yl)-methyl]-benzimidazol und

C = 1-[(1H-Tetrazol-5-yl)-methyl]-2-phenyl-5-[(2-n-propyl-4-methyl-6-(1-methyl-benzimidazol-2-yl)-benzimidazol-1-yl)-methyl]-benzimidazol

auf ihre biologischen Wirkungen wie folgt untersucht:

Methodenbeschreibung Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37 °C mit 50 pM [$125^I$]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis C zeigen in dem beschriebenen Test folgende $IC_{50}$-Werte:

| Substanz | $IC_{50}$ [nM] |
|---|---|
| A | 120 |
| B | 23 |
| C | 2,3 |

Desweiteren konnte an Ratten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, der Bulimie, sowie von Störungen kognitiver Funktionen.

Die zur Erzielung einer entsprechenden Wirkung am Erwachsenen erforderliche Dosierung beträgt zweckmäßigerweise bei intravenöser Gabe 20 bis 100 mg, vorzugsweise 30 bis 70 mg, und bei oraler Gabe 50 bis 200 mg, vorzugsweise 75 bis 150 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, Diuretika und/oder Kalzium-Antagonisten, zusammen mit einem oder mehreren inerten üblichen Trägerstoffen und/oder Verdünnungsmitteln, z.B. mit Maisstärke, Milchzukker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin und Nitrendipin in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

Beispiel 1

Gemisch aus 1-Hydroxycarbonylmethyl-2-phenyl-5-[(2-n-butyl-benzimidazol-1-yl)-methyl]-benzimidazol und 1-Hydroxycarbonylmethyl-2-phenyl-6-[(2-n-butyl-benzimidazol-1-yl)-methyl]-benzimidazol

a) 1-(4-Amino-3-nitro-benzyl)-2-n-butyl-benzimidazol

34,5 g (109 mMol) 1-(4-Chlor-3-nitro-benzyl)-2-n-butyl-benzimidazolwerden mit 200 ml flüssigem Ammoniak versetzt und im Autoklaven 8 Stunden lang auf 120 °C erhitzt. Nach dem Abdampfen des überschüssigen Ammoniaks wird das so erhaltene Rohprodukt aus ca. 250 ml Methanol umkristallisiert.
Ausbeute: 31,2 g (96 % der Theorie), $R_f$-Wert: 0,44 (Kieselgel; Methylenchlorid/Methanol = 19:1)

b) 1-(4-Benzoylamino-3-nitro-benzyl)-2-n-butyl-benzimidazolhydrochlorid

Eine Lösung aus 28,0 g (86 mMol) 1-(4-Amino-3-nitro-benzyl)-2-n-butyl-benzimidazol und 16,9 g (120 mMol) Benzoylchlorid in 500 ml Chlorbenzol wird 16 Stunden lang zum Rückfluß erhitzt, anschließend das Chlorbenzol abdestillierr und das so erhaltene Rohprodukt aus ca. 300 ml Ethanol/Diethylether (2:1) umkristallisiert.
Ausbeute: 26,0 g (71 % der Theorie),
$R_f$-Wert: 0,59 (Kieselgel; Methylenchlorid/Methanol = 19:1)
$C_{25}H_{24}N_4O_3$ x HCl (428,49)

| | | | | | | |
|---|---|---|---|---|---|---|
| Ber.: | C | 64,58 | H | 5,42 | N | 12,05 |
| Gef.: | | 64,34 | | 5,33 | | 12,17 |

c) 1-(4-Benzoylamino-3-amino-benzyl)-2-n-butyl-benzimidazol

9,0 g (19,4 mMol) 1-(4-Benzoylamino-3-nitro-benzyl)-2-n-butyl-benzimidazol-hydrochlorid, gelöst in 500 ml Methanol, werden mit 1 g 10%igem Palladium/Kohle versetzt und bei Raumtemperatur mit 5 bar Wasserstoffdruck hydriert. Nach vollständiger Reaktion wird vom Katalysator abfiltriert und das Filtrat zur Trockne eingeengt. Das so erhaltene Produkt wird ohne weitere Reinigung weiter umgesetzt.
Ausbeute: 7,7 g (99,5 % der Theorie),
$R_f$-Wert: 0,38 (Kieselgel; Methylenchlorid/Methanol = 19:1)

d) 2-Phenyl-5-[(2-n-butyl-benzimidazol-1-yl)-methyl]-benzimidazol

Eine Lösung von 7,7 g (19,3 mMol) 1-(4-Benzoylamino-3-aminobenzyl)-2-n-butyl-benzimidazol in 100 ml Eisessig wird 2 Stunden lang auf 100 °C erhitzt, anschließend ca. 95 ml Eisessig abdestilliert, der Rückstand mit ca. 150 ml Wasser versetzt und mit konzentriertem Ammoniak alkalisch gestellt. Das ausgefallene Rohprodukt wird abgesaugt und durch Säulenchromatographie (500 g Kieselgel; Laufmittel: Methylenchlorid/Methanol : 50:1) gereinigt.
Ausbeute: 5,4 g (73 % der Theorie),
$R_f$-Wert: 0,36 (Kieselgel; Methylenchlorid/Methanol = 19:1)
$C_{25}H_{24}N_4$ (380,49)

| | | | | | | |
|---|---|---|---|---|---|---|
| Ber.: | C | 78,92 | H | 6,36 | N | 14,72 |
| Gef.: | | 78,68 | | 6,43 | | 14,58 |

e) Gemisch aus 1-Ethoxycarbonylmethyl-2-phenyl-5-[(2-n-butyl-benzimidazol-1-yl)-methyl]-benzimidazol und 1-Ethoxycarbonylmethyl-2-phenyl-6-[(2-n-butyl-benzimidazol-1-yl)-methyl]-benzimidazol

Eine Lösung von 810 mg (7,2 mMol) Kalium-tert.butylat, 2,7 g (7,1 mMol) 2-Phenyl-5-[(2-n-butyl-benzimidazol-1-yl)-methyl]-benzimidazol und 1,2 g (7,2 mMol) Bromessigsäureethylester in 50 ml Dimethylsulfoxid wird vier Stunden lang bei Raumtemperatur gerührt, dann mit ca. 300 ml Wasser versetzt und

dreimal mit je ca. 40 ml Essigester extrahiert. Die organischen Extrakte werden mit ca. 30 ml Wasser gewaschen, getrocknet und eingedampft. Das so erhaltene rohe Isomerengemisch wird ohne weitere Reinigung weiter umgesetzt.
Ausbeute: 3,3 g (100 % der Theorie),
$R_f$-Wert: 0,42 (Kieselgel; Methylenchlorid/Methanol = 19:1)

f) Gemisch bestehend aus 1-Hydroxycarbonylmethyl-2-phenyl-5-[(2-n-butyl-benzimidazol-1-yl)-methyl]-benzimidazol und 1-Hydroxycarbonylmethyl-2-phenyl-6-[(2-n-butyl-benzimidazol-1-yl)-methyl]-benzimidazol

3,3 g (7,0 mMol) des gemäß Beispiel 1e erhaltenen Gemisches werden in einer Lösung von 1,2 g (30 mMol) Natriumhydroxid in 20 ml Wasser und 50 ml Ethanol bei Raumtemperatur 4 Stunden lang gerührt. Dann wird das Ethanol abdestilliert, der Rückstand mit ca. 30 ml Wasser verdünnt, mit Essigsäure angesäuert und das ausgefallene Rohprodukt abgesaugt. Die Reinigung erfolgt durch Säulenchromatographie (300 g Kieselgel; Laufmittel = Methylenchlorid/Methanol = 9:1).
Ausbeute: 2,0 g (65 % der Theorie),
Schmelzpunkt: amorph
$R_f$-Wert: 0,24 (Kieselgel; Methylenchlorid/Methanol = 9:1)
$C_{27}H_{26}N_4O_2$ (438,53)

| | | | | | | |
|---|---|---|---|---|---|---|
| Ber.:<br>Gef.: | C | 73,95<br>73,74 | H | 5,98<br>6,05 | N | 12,78<br>12,67 |

Beispiel 2

1-Hydroxycarbonylmethyl-2-phenyl-5-[(2-ethyl-5,7-dimethylimidazo[4,5-b]pyridin-3-yl)-methyl]-benzimidazol-trihydrat

Hergestellt analog Beispiel 1 aus 1-Ethoxycarbonylmethyl-2-phenyl-5-[(2-ethyl-5,7-dimethyl-imidazo[4,5-b]pyridin-3-yl)-methyl]-benzimidazol und wäßriger Natronlauge in Ethanol.
Ausbeute: 48 % der Theorie,
Schmelzpunkt: 170-172 °C
$R_f$-Wert: 0,29 (Kieselgel; Methylenchlorid/Methanol = 8:2)
$C_{26}H_{25}N_5O_2$ x 3 $H_2O$ (439,52)

| | | | | | | |
|---|---|---|---|---|---|---|
| Ber.:<br>Gef.: | C | 63,27<br>63,47 | H | 6,33<br>6,12 | N | 14,19<br>14,28 |

Beispiel 3

1-Hydroxycarbonylmethyl-2-phenyl-6-[(2-ethyl-5,7-dimethyl-imidazo[4,5-b]pyridin-3-yl)-methyl]-benzimidazol-semihydrat

Hergestellt analog Beispiel 1 aus 1-Ethoxycarbonylmethyl-2-phenyl-5-[(2-ethyl-5,7-dimethyl-imidazo[4,5-b]pyridin-3-yl)-methyl]-benzimidazol und wäßriger Natronlauge in Ethanol.
Ausbeute: 57 % der Theorie,
Schmelzpunkt: 256-257 °C
$R_f$-Wert: 0,30 (Kieselgel; Methylenchlorid/Methanol = 8:2)
$C_{26}H_{25}N_5O_2$ x 1/2 $H_2O$ (439,52)

| | | | | | | |
|---|---|---|---|---|---|---|
| Ber.:<br>Gef.: | C | 69,62<br>69,50 | H | 5,84<br>5,91 | N | 15,61<br>15,77 |

Beispiel 4

Gemisch aus 1-Hydroxycarbonylmethyl-2-cyclopropyl-5-[(2-n-propyl-4-methyl-benzimidazol-1-yl)-methyl]-benzimidazol Natriumsalz x Natriumacetat x 2 $H_2O$ und 1-Hydroxycarbonylmethyl-2-cyclopropyl-6-[(2-n-propyl-4-methyl-benzimidazol-1-yl)-methyl]-benzimidazol Natriumsalz x Natriumacetat x 2 $H_2O$

Hergestellt analog Beispiel 1 aus einem Gemisch aus 1-Ethoxycarbonylmethyl-2-cyclopropyl-5-[(2-n-propyl-4-methyl-benzimidazol-1-yl)-methyl]-benzimidazol und 1-Ethoxycarbonylmethyl-2-cyclopropyl-6-[(2-n-propyl-4-methyl-benzimidazol-1-yl)-methyl]-benzimidazol und wäßriger Natronlauge in Ethanol.
Ausbeute: 80 % der Theorie,
Schmelzpunkt: amorph
$R_f$-Wert: 0,25 (Kieselgel; Methylenchlorid/Methanol = 8:2)
$C_{24}H_{25}N_4O_2Na$ x $CH_3COONa$ x 2 $H_2O$ (542,55)

| | | | | | | |
|---|---|---|---|---|---|---|
| Ber.:<br>Gef.: | C | 57,55<br>57,63 | H | 5,95<br>5,95 | N | 10,33<br>10,18 |

Beispiel 5

1-Hydroxycarbonylmethyl-2-phenyl-6-[(2-n-propyl-4-methyl-6-(1-methyl-benzimidazol-2-yl)-benzimidazol-1-yl)-methyl]-benzimidazol

Hergestellt analog Beispiel 1 aus 1-Ethoxycarbonylmethyl-2-phenyl-6-[(2-n-propyl-4-methyl-6-(1-methyl-benzimidazol-2-yl)-benzimidazol-1-yl)-methyl]-benzimidazol und wäßriger Natronlauge in Ethanol.
Ausbeute: 10 % der Theorie,
Schmelzpunkt: ab 250 °C (Zers.)
$R_f$-Wert: 0,14 (Kieselgel; Methylenchlorid/Methanol = 9:1)
$C_{35}H_{32}N_6O_2$ (568,68)

| | | | | | | |
|---|---|---|---|---|---|---|
| Ber.:<br>Gef.: | C | 73,92<br>73,73 | H | 5,67<br>5,71 | N | 14,78<br>14,86 |

Beispiel 6

1-Hydroxycarbonylmethyl-2-phenyl-5-[(2-n-propyl-4-methyl-6-(1-methyl-benzimidazol-2-yl)-benzimidazol-1-yl)-methyl]-benzimidazol

Hergestellt analog Beispiel 1 aus 1-Ethoxycarbonylmethyl-2-phenyl-5-[(2-n-propyl-4-methyl-6-(1-methyl-benzimidazol-2-yl)-benzimidazol-1-yl)-methyl]-benzimidazol und wäßriger Natronlauge in Ethanol.
Ausbeute: 30 % der Theorie,
Schmelzpunkt: ab 235 °C (Zers.)
$R_f$-Wert: 0,34 (Kieselgel; Methylenchlorid/Methanol = 9:1)
$C_{35}H_{32}N_6O_2$ (568,68)

| | | | | | | |
|---|---|---|---|---|---|---|
| Ber.:<br>Gef.: | C | 73,92<br>73,75 | H | 5,67<br>5,71 | N | 14,78<br>14,59 |

Beispiel 7

1-[(1H-Tetrazol-5-yl)-methyl]-2-phenyl-5-[(2-n-butyl-benzimidazol-1-yl)-methyl]-benzimidazol

a) Gemisch aus 1-Cyanomethyl-2-phenyl-5-[(2-n-butyl-benzimidazol-1-yl)-methyl]-benzimidazol und 1-Cyanomethyl-2-phenyl-6-[(2-n-butyl-benzimidazol-1-yl)-methyl]-benzimidazol

Eine Lösung von 2,1 g (5,5 mMol) 2-Phenyl-5-[(2-n-butyl-benzimidazol-1-yl)-methyl]-benzimidazol und 530 mg Chloracetonitril in 30 ml Dimethylsulfoxid wird mit 1,9 g (11 mMol) Kaliumcarbonat-dihydrat versetzt und 2 Stunden lang bei 60 °C gerührt. Nach dem Abkühlen werden ca. 100 ml Wasser zugegeben, dann das ausgefallene Rohprodukt abgesaugt und durch Säulenchromatographie gereinigt (300 g Kieselgel; Laufmittel: Methylenchlorid/Methanol = 30:1).
Ausbeute: 1,2 g (52 % der Theorie),
$R_f$-Wert: 0,68 (Kieselgel; Methylenchlorid/Methanol = 19:1)

b) 1-[(1H-Tetrazol-5-yl)-methyl]-2-phenyl-5-[(2-n-butyl-benzimidazol-1-yl)-methyl]-benzimidazol

1,2 g (2,9 mMol) des gemäß Beispiel 7a erhaltenen Gemisches werden in 25 ml Dimethylformamid gelöst, mit 3,1 g (57,2 mMol) Ammoniumchlorid und 3,7 g (57,2 mMol) Natriumazid versetzt und 3 Stunden lang bei 140 °C gerührt. Dann wird mit ca. 80 ml Wasser versetzt und nach 2 Stunden Rühren bei Raumtemperatur das ausgefallene Rohprodukt abgesaugt und getrocknet. Man erhält so 1,2 g des Isomerengemisches der analogen Tetrazol-Verbindungen. Durch fraktionierende Kristallisation aus Methanol wird daraus das 5-Isomer in reiner Form gewonnen, während das 6-Isomer in angereichterter Form in der Mutterlauge zurückbleibt.
Ausbeute: 0,54 g (41 % der Theorie),
Schmelzpunkt: ab 195 °C sintern
$R_f$-Wert: 0,40 (Kieselgel; Methylenchlorid/Methanol = 8:2)
$C_{27}H_{26}N_8$ (462,57)

| | | | | | | |
|---|---|---|---|---|---|---|
| Ber.:<br>Gef.: | C | 70,11<br>69,93 | H | 5,67<br>5,77 | N | 24,23<br>24,10 |

Beispiel 8

1-[(1H-Tetrazol-5-yl)-methyl]-2-phenyl-6-[(2-n-butyl-benzimidazol-1-yl)-methyl]-benzimidazol

Hergestellt durch Einengen der gemäß Beispiel 7b erhaltenen Mutterlauge und anschließende Kristallisation aus Methanol.
Ausbeute: 0,40 g (30 % der Theorie),
Schmelzpunkt: ab 140 °C (Zers.)
$R_f$-Wert: 0,33 (Kieselgel; Methylenchlorid/Methanol = 8:2)
$C_{27}H_{26}N_8$ (462,57)

| | | | | | | |
|---|---|---|---|---|---|---|
| Ber.:<br>Gef.: | C | 70,11<br>69,96 | H | 5,67<br>5,89 | N | 24,23<br>24,05 |

Beispiel 9

Gemisch aus 1-[(1H-Tetrazol-5-yl)-methyl]-2-cyclopropyl-5-[(2-n-propyl-4-methyl-benzimidazol-1-yl)-methyl]-benzimidazol und
1-[(1H-Tetrazol-5-yl)-methyl]-2-cyclopropyl-6-[(2-n-propyl-4-methyl-benzimidazol-1-yl)-methyl]-benzimidazol

Hergestellt analog Beispiel 7b aus 1-Cyanomethyl-2-cyclopropyl-5-[(2-n-propyl-4-methyl-benzimidazol-1-yl)-methyl]-benzimidazol und 1-Cyanomethyl-2-cyclopropyl-6-[(2-n-propyl-4-methyl-benzimidazol-1-yl)-methyl]-benzimidazol und Natriumazid in Dimethylformamid.
Ausbeute: 52 % der Theorie,
Schmelzpunkt: 203-205 °C
$R_f$-Wert: 0,50 (Kieselgel; Methylenchlorid/Methanol = 8:2)
$C_{24}H_{26}N_8$ (426,53)

| | | | | | | |
|---|---|---|---|---|---|---|
| Ber.:<br>Gef.: | C | 67,58<br>67,47 | H | 6,14<br>6,40 | N | 26,27<br>26,45 |

Beispiel 10

Gemisch aus 1-[(1H-Tetrazol-5-yl)-methyl]-2-phenyl-5-[(2-ethyl-5,7-dimethyl-imidazol[4,5-b]pyridin-3-yl)-methyl]-benzimidazol und
1-[(1H-Tetrazol-5-yl)-methyl]-2-phenyl-6-[(2-ethyl-5,7-dimethyl-imidazol[4,5-b]pyridin-3-yl)-methyl]-benzimidazol

Hergestellt analog Beispiel 7b aus einem Gemisch aus 1-Cyanomethyl-2-phenyl-5-[(2-ethyl-5,7-dimethyl-imidazo[4,5-b]pyridin-3-yl)-methyl]-benzimidazol und 1-Cyanomethyl-2-phenyl-6-[(2-ethyl-5,7-dimethyl-imidazol[4,5-b]pyridin-3-yl)-methyl]-benzimidazol und Natriumazid in Dimethylformamid.
Ausbeute: 37 % der Theorie,
Schmelzpunkt: 172-174 °C
$R_f$-Wert: 0,16 (Kieselgel; Methylenchlorid/Methanol = 9:1)
$C_{26}H_{25}N_9$ (463,56)
Massenspektrum: m/e = 463

Beispiel 11

1-[(1H-Tetrazol-5-yl)-methyl]-2-phenyl-5-[(2-n-propyl-4-methyl-6-(1-methyl-benzimidazol-2-yl)-benzimidazol-1-yl)-methyl]-benzimidazol

Hergestellt analog Beispiel 7b aus 1-Cyanomethyl-2-phenyl-5-[(2-n-propyl-4-methyl-6-(1-methyl-benzimidazol-2-yl)-benzimidazol-1-yl)-methyl]-benzimidazol und Natriumazid in Dimethylformamid.
Ausbeute: 11 % der Theorie,
Schmelzpunkt: ab 203 °C (Zers.)
$R_f$-Wert: 0,34 (Kieselgel; Methylenchlorid/Methanol = 9:1)
$C_{35}H_{32}N_{10}$ (592,72)

| | | | | | | |
|---|---|---|---|---|---|---|
| Ber.:<br>Gef.: | C | 70,92<br>70,76 | H | 5,44<br>5,72 | N | 23,63<br>23,57 |

Beispiel 12

1-[(1H-Tetrazol-5-yl)-methyl]-2-phenyl-6-[(2-n-propyl-4-methyl-6-(1-methyl-benzimidazol-2-yl)-benzimidazol-1-yl)-methyl]-benzimidazol-trihydrat

Hergestellt analog Beispiel 7b aus 1-Cyanomethyl-2-phenyl-6-[(2-n-propyl-4-methyl-6-(1-methyl-benzimidazol-2-yl)-benzimidazol-1-yl)-methyl]-benzimidazol und Natriumazid in Dimethylformamid.
Ausbeute: 12 % der Theorie,
Schmelzpunkt: ab 205 °C (Zers.)
$R_f$-Wert: 0,11 (Kieselgel; Methylenchlorid/Methanol = 9:1)
$C_{35}H_{32}N_{10}$ x 3 $H_2O$ (592,72)

| | | | | | | |
|---|---|---|---|---|---|---|
| Ber.: | C | 65,00 | H | 5,92 | N | 21,65 |
| Gef.: | | 65,21 | | 5,76 | | 21,68 |

Bei den nachfolgenden pharmazeutischen Anwendungsbeispielen kann als Wirksubstanz jede geeignete Verbindung der Formel I, insbesondere diejenigen in denen $R_b$ eine Carboxy- oder 1H-Tetrazolylgruppe darstellt, eingesetzt werden:

Beispiel I

| Ampullen, enthaltend 50 mg Wirkstoff pro 5 ml | |
|---|---|
| Wirkstoff | 50 mg |
| $KH_2PO_4$ | 2 mg |
| $Na_2HPO_4$ x $2H_20$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel IV

| Dragees, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45 °C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 ° C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40 ° C wird die gemahlene Wirksubstanz in der Schmelze homogen dispergiert. Es wird auf 38 ° C abgekühlt und in schwach vorgekühlte Suppositorienformen ausgegossen.

**Patentansprüche**

**1.** Substituierte Benzimidazolylderivate der allgemeinen Formel

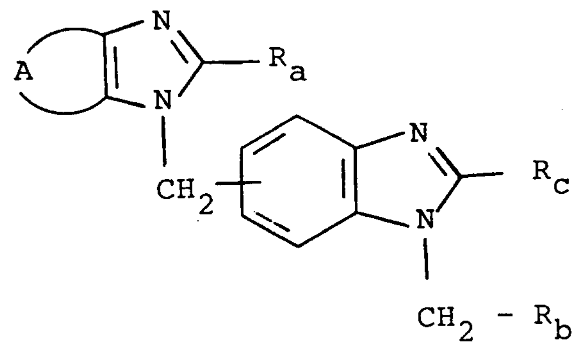

, (I)

in der
A eine 1,4-Butadienylengruppe, die durch die Reste $R_1$ und $R_2$ substituiert ist und in der zusätzlich eine unsubstituierte Methingruppe durch ein Stickstoffatom ersetzt sein kann, wobei
$R_1$ ein Wasserstoff-, Fluor-, Chlor- oder Bromatom, eine Trifluormethylgruppe oder eine Alkylgruppe mit 1 bis 3 Kohlenstoffatomen und
$R_2$ ein Wasserstoffatom, eine Alkylgruppe mit 1 bis 3 Kohlenstoffatomen,
eine Carboxygruppe, eine Alkoxycarbonylgruppe mit insgesamt 2 bis 6 Kohlenstoffatomen,
eine Alkoxygruppe mit 2 bis 5 Kohlenstoffatomen, die in 2-, 3-, 4- oder 5-Position durch eine Imidazolyl-, Benzimidazolyl- oder Tetrahydrobenzimidazolylgruppe
substituiert ist,
eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können,
eine Phthalimino- oder Homophthaliminogruppe, wobei eine Carbonylgruppe in einer Phthaliminogruppe durch eine Methylengruppe ersetzt sein kann,
eine 5-, 6- oder 7-gliedrige Alkyleniminogruppe, in welcher eine Methylengruppe durch eine Carbonyl- oder Sulfonylgruppe ersetzt sein kann,
eine Glutarsäureiminogruppe, in der die n-Propylengruppe durch ein oder zwei Alkylgruppen mit jeweils 1 bis 3 Kohlenstoffatomen oder durch eine Tetramethylen- oder Pentamethylengruppe substituiert sein kann,
eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
eine gegebenenfalls in 1-Stellung durch eine Alkylgruppe mit 1 bis 6 Kohlenstoffatomen oder eine Cycloalkylgruppe mit 3 bis 7 Kohlenstoffatomen substituierte Benzimidazol-2-yl-gruppe, wobei der Phenylkern in einer vorstehend erwähnten Benzimidazol-2-ylgruppe zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-, Imidazo[1,2-a]pyrimidin-2-yl-, Imidazo[4,5-b]pyridin-2-yl-, Imidazo[4,5-c]pyridin-2-yl-, Imidazo[1,2-c]pyrimidin-2-yl-, Imidazo[1,2-a]-pyrazin-2-yl-, Imidazo[1,2-b]pyridazin-2-yl-, Imidazo[4,5-c]pyridin-2-yl-, Purin-8-yl-, Imidazo[4,5-b]-pyrazin-2-yl-, Imidazo[4,5-c]pyridazin-2-yl- oder Imidazo[4,5-d]pyridazin-2-yl-gruppe
einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonyl- oder Sulfonylgruppe ersetzt sein kann,
eine gegebenenfalls in 2-Stellung durch eine Alkylgruppe mit 1 bis 6 Kohlenstoffatomen oder durch eine Phenylgruppe substituierte Imidazol-4-yl-Gruppe, die in 1-Stellung durch eine Alkylgruppe mit 1 bis 7 Kohlenstoffatomen, die in 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxidothiomorpholinocarbonylgruppe substituiert sein kann, durch eine Alkylgruppe mit 2 bis 4 Kohlenstoffatomen, die in 2-, 3- oder 4-Stellung durch eine Hydroxy-, Alkoxy- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine gegebenenfalls durch Fluor- oder Chloratome, durch Trifluormethyl-, Methyl- oder Methoxygruppen mono- oder disubstituierte

Phenylgruppe substituiert ist, durch eine durch zwei Phenylgruppen substituierte Alkylgruppe oder durch eine Cycloalkylgruppe mit 3 bis 7 Kohlenstoffatomen substituiert ist, wobei, soweit nichts anderes erwähnt wurde, die vorstehend erwähnten Alkyl- und Alkoxyteile jeweils 1 bis 3 Kohlenstoffatome enthalten können,
eine Carboxy-, Aminocarbonyl-, Alkylaminocarbonyl- oder Dialkylaminogruppe, in denen der Alkylteil jeweils 1 bis 6 Kohlenstoffatome enthalten kann, oder eine Gruppe, die in-vivo metabolisch in eine Carboxygruppe umgewandelt wird, oder
eine $R_5$-$NR_4$-CO-$NR_3$-Gruppe, in der
$R_3$ ein Wasserstoffatom, eine Alkylgruppe mit 1 bis 5 Kohlenstoffatomen, eine Cyclohexyl- oder Benzylgruppe,
$R_4$ ein Wasserstoffatom, eine Alkylgruppe mit 1 bis 6 Kohlenstoffatomen, eine Allyl-, Cyclohexyl-, Benzyl- oder Phenylgruppe,
$R_5$ ein Wasserstoffatom oder eine Alkylgruppe mit 1 bis 3 Kohlenstoffatomen oder
$R_4$ und $R_5$ zusammen mit dem dazwischen liegenden Stickstoffatom eine unverzweigte Cycloalkyleniminogruppe mit 4 bis 6 Kohlenstoffatomen oder eine Morpholinogruppe oder
$R_3$ und $R_4$ zusammen eine Alkylengruppe mit 2 oder 3 Kohlenstoffatomen darstellen,
$R_a$ eine Alkylgruppe mit 1 bis 5 Kohlenstoffatomen, eine Cycloalkylgruppe mit 3 bis 5 Kohlenstoffatomen, eine Alkoxy-, Alkylthio- oder Alkylaminogruppe mit jeweils 1 bis 3 Kohlenstoffatomen in jedem Alkylteil,
$R_b$ eine Carboxy-, Cyano-, Hydroxysulfonyl-, 1H-Tetrazolyl-, 1-Triphenylmethyl-tetrazolyl- oder 2-Triphenylmethyl-tetrazolylgruppe, eine Gruppe, die in-vivo metabolisch in eine Carboxygruppe umgewandelt wird, eine Alkancarbonylaminosulfonyl-, Benzoylaminosulfonyl-, Alkansulfonylaminocarbonyl-, Trifluormethansulfonylaminocarbonyl- oder Phenylsulfonylaminocarbonylgruppe, wobei in den vorstehend erwähnten Gruppen die Alkyl- und Alkoxyteile jeweils 1 bis 4 Kohlenstoffatome enthalten können, und
$R_c$ eine Alkylgruppe, eine Cycloalkylgruppe mit 3 bis 6 Kohlenstoffatomen oder eine Phenylgruppe, die durch ein Fluor-, Chlor- oder Bromatom, durch eine Hydroxy-, Alkoxy- oder Alkylgruppe mono- oder disubstituiert sein kann, wobei die Substituenten gleich oder verschieden sein können und die in den vorstehend erwähnten Gruppen erwähnten Alkyl- und Alkoxyteile jeweils 1 bis 6 Kohlenstoffatome enthalten können, bedeuten,
deren Gemische von Stellungsisomeren und deren Salze.

**2.** Substituierte Benzimidazolylderivate der allgemeinen Formel I gemäß Anspruch 1, in der A, $R_a$ und $R_c$ wie im Anspruch 1 definiert sind und
$R_b$ eine Carboxy- oder 1H-Tetrazolylgruppe oder eine Gruppe der Formeln

- CO - OR',
- CO - O - (HCR'') - O - CO - R''' und
- CO - O - (HCR'') - O - CO - OR'''

darstellt, in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten,
deren Gemische von Stellungsisomeren und deren Salze.

**3.** Substituierte Benzimidazolylderivate der allgemeinen Formel I gemäß Anspruch 1, in denen
A eine 1,4-Butadienylengruppe, die durch die Reste $R_1$ und $R_2$ substituiert ist und in der zusätzlich die Methingruppe in Position 7 des so gebildeten Benzimidazols durch ein Stickstoffatom ersetzt sein kann, wobei
$R_1$ ein Wasserstoffatom oder in 4-Stellung ein Fluor-, Chlor- oder Bromatom, eine Trifluormethylgruppe oder eine Alkylgruppe mit 1 bis 3 Kohlenstoffatomen und
$R_2$ ein Wasserstoffatom,
eine Alkylgruppe mit 1 bis 3 Kohlenstoffatomen,

in 6-Stellung eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können,
in 6-Stellung eine Phthalimino- oder Homophthaliminogruppe, wobei eine Carbonylgruppe in einer Phthaliminogruppe durch eine Methylengruppe ersetzt sein kann,
in 6-Stellung eine 5-, 6- oder 7-gliedrige Alkyleniminogruppe, in welcher eine Methylengruppe durch eine Carbonyl- oder Sulfonylgruppe ersetzt ist,
in 6-Stellung eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
in 6-Stellung eine gegebenenfalls in 1-Stellung durch eine Alkylgruppe mit 1 bis 6 Kohlenstoffatomen oder eine Cycloalkylgruppe mit 3 bis 7 Kohlenstoffatomen substituierte Benzimidazol-2-yl-gruppe, wobei der Phenylkern in einer vorstehend erwähnten Benzimidazol-2-ylgruppe zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-, Imidazo[1,2-a]pyrimidin-2-yl- oder Imidazo[4,5-b]pyridin-2-yl-gruppe,
in 6-Stellung eine Imidazol-4-yl-Gruppe, die in 1-Stellung durch eine Alkylgruppe mit 1 bis 7 Kohlenstoffatomen, die in 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxido-thiomorpholinocarbonylgruppe substituiert sein kann, durch eine Alkylgruppe mit 2 bis 4 Kohlenstoffatomen,
die in 2-, 3- oder 4-Stellung durch eine Hydroxy-, Alkoxy- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine gegebenenfalls durch Fluor- oder Chloratome, durch Trifluormethyl-, Methyl- oder Methoxygruppen mono- oder disubstituierte Phenylgruppe substituiert ist, durch eine durch zwei Phenylgruppen substituierte Alkylgruppe oder durch eine Cycloalkylgruppe mit 3 bis 7 Kohlenstoffatomen substituiert ist, wobei, soweit nichts anderes erwähnt wurde, die vorstehend erwähnten Alkyl- und Alkoxyteile jeweils 1 bis 3 Kohlenstoffatome enthalten können,
in 7-Stellung eine Carboxy-, Aminocarbonyl-, Alkylaminocarbonyl- oder Dialkylaminogruppe, in denen der Alkylteil jeweils 1 bis 6 Kohlenstoffatome enthalten kann, oder eine Gruppe, die in-vivo metabolisch in eine Carboxygruppe umgewandelt wird, oder
in 6-Stellung eine $R_5$-$NR_4$-CO-$NR_3$-Gruppe, in der

$R_3$ ein Wasserstoffatom, eine Alkylgruppe mit 1 bis 5 Kohlenstoffatomen, eine Cyclohexyl- oder Benzylgruppe,
$R_4$ ein Wasserstoffatom, eine Alkylgruppe mit 1 bis 6 Kohlenstoffatomen, eine Allyl-, Cyclohexyl-, Benzyl- oder Phenylgruppe,
$R_5$ ein Wasserstoffatom oder eine Alkylgruppe mit 1 bis 3 Kohlenstoffatomen oder
$R_4$ und $R_5$ zusammen mit dem dazwischen liegenden Stickstoffatom eine unverzweigte Cycloalkyleniminogruppe mit 4 bis 6 Kohlenstoffatomen oder eine Morpholinogruppe oder
$R_3$ und $R_4$ zusammen eine Alkylengruppe mit 2 oder 3 Kohlenstoffatomen darstellen,

$R_a$ eine Alkylgruppe mit 2 bis 4 Kohlenstoffatomen, eine Cycloalkylgruppe mit 3 oder 4 Kohlenstoffatomen oder eine Alkoxygruppe mit 2 oder 3 Kohlenstoffatomen,
$R_b$ eine Gruppe, die in-vivo in eine Carboxygruppe umgewandelt wird, eine Carboxy- oder 1H-Tetrazolylgruppe und
$R_c$ eine Alkylgruppe mit 1 bis 3 Kohlenstoffatomen, eine Cycloalkylgruppe mit 3 bis 6 Kohlenstoffatomen oder eine Phenylgruppe bedeuten, wobei die Gruppe, die in-vivo metabolisch in eine Carboxygruppe umgewandelt wird, jeweils wie im Anspruch 2 definiert ist,
deren Gemische von Stellungsisomeren und deren Salze.

**4.** Substituierte Benzimidazolylderivate der allgemeinen Formel I gemäß Anspruch 1, in denen
A eine 1,4-Butadienylengruppe, in der die Methingruppe in den Positionen 4 und 6 des so gebildeten Benzimidazols durch Methylgruppen substituiert sind und die Methingruppe in Position 7 durch ein Stickstoffatom ersetzt ist, oder
A eine 1,4-Butadienylengruppe, die durch die Reste $R_1$ und $R_2$ substituiert ist, wobei
$R_1$ ein Wasserstoffatom oder in 4-Stellung eine Methylgruppe und
$R_2$ in 6-Stellung eine 1-Methyl-benzimidazol-2-yl-Gruppe darstellen,
$R_a$ eine n-Alkylgruppe mit 2 bis 4 Kohlenstoffatomen,
$R_b$ eine Carboxy- oder 1H-Tetrazolylgruppe und

$R_c$ eine Alkylgruppe mit 1 bis 3 Kohlenstoffatomen, eine Cycloalkylgruppe mit 3 bis 6 Kohlenstoffatomen oder eine Phenylgruppe bedeuten,
deren Gemische von Stellungsisomeren und deren Salze.

**5.** Folgende Verbindungen der allgemeinen Formel I gemäß Anspruch 1:
1-Hydroxycarbonylmethyl-2-phenyl-5-[(2-ethyl-4,6-dimethylimidazo[4,5-b]pyridin-1-yl)-methyl]-benzimidazol,
1-Hydroxycarbonylmethyl-2-phenyl-6-[(2-n-propyl-4-methyl-6-(1-methyl-benzimidazol-2-yl)-benzimidazol-1-yl)-methyl]-benzimidazol und
1-[(1H-Tetrazol-5-yl)-methyl]-2-phenyl-5-[(2-n-propyl-4-methyl-6-(1-methyl-benzimidazol-2-yl)-benzimidazol-1-yl)-methyl]-benzimidazol,
deren Gemische von Stellungsisomeren und deren Salze.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindesters einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels mit Angiotensin-antagonistischer Wirkung.

**9.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der substituierten Benzimidazolylderivate der allgemeinen Formel I gemäß den Ansprüchen 1 bis 6, dadurch gekennzeichnet, daß
a) ein Benzimidazol der allgemeinen Formel

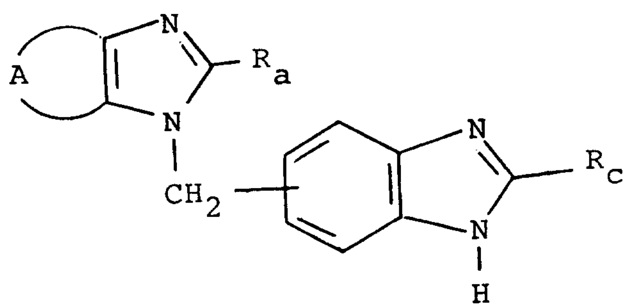

,(II)

in der
$R_a$, $R_c$ und A wie in den Ansprüchen 1 bis 5 definiert sind, mit einer Verbindung der allgemeinen Formel

$Z_1$ - $CH_2$ - $R_b$ ,(III)

in der
$R_b$ mit Ausnahme der 1H-Tetrazolylgruppe wie in den Ansprüchen 1 bis 5 definiert ist und
$Z_1$ eine nukleophile Austrittsgruppe darstellt, umgesetzt und gegebenenfalls anschließend die 1-Triphenylmethylgruppe von einer so erhaltenen Triphenylmethyl-tetrazolylverbindung abgespalten oder ein so erhaltener Ester hydrolysiert wird oder
b) zur Herstellung einer Verbindung der allgemeinen Formel I, in der $R_b$ eine Carboxygruppe darstellt, eine Verbindung der allgemeinen Formel

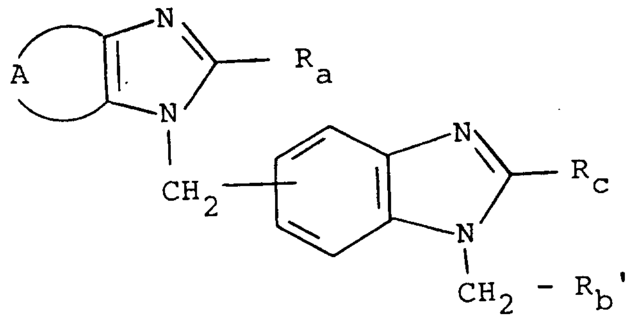

,(IV)

in der

$R_a$, $R_c$ und A wie in den Ansprüchen 1 bis 5 definiert sind und $R_b$' eine mittels Hydrolyse, Thermolyse oder Hydrogenolyse in eine Carboxygruppe überführbare Gruppe darstellt, in eine entsprechende Carboxyverbindung übergeführt wird oder

c) zur Herstellung einer Verbindung der allgemeinen Formel I, in der $R_b$ eine 1H-Tetrazolylgruppe darstellt, ein Schutzrest von einer Verbindung der allgemeinen Formel

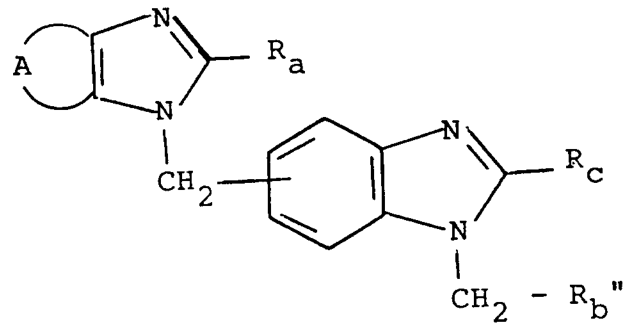

,(V)

in der

$R_a$, $R_c$ und A wie in den Ansprüchen 1 bis 5 definiert sind und $R_b$" eine in 1- oder 2-Stellung durch einen Schutzrest geschützte 1H-Tetrazolylgruppe darstellt, abgespalten wird oder

d) zur Herstellung einer Verbindung der allgemeinen Formel I, in der $R_b$ eine 1H-Tetrazolylgruppe darstellt, eine Verbindung der allgemeinen Formel

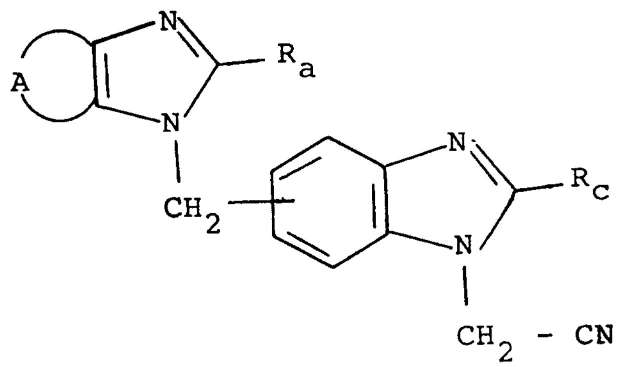

,(VI)

in der

$R_a$, $R_c$ und A wie in den Ansprüchen 1 bis 5 definiert sind, mit Stickstoffwasserstoffsäure oder deren Salzen umgesetzt wird oder

e) zur Herstellung einer Verbindung der allgemeinen Formel I, in der $R_2$ eine $R_5$-$NR_4$-$CONR_3$-Gruppe darstellt, eine Verbindung der allgemeinen Formel

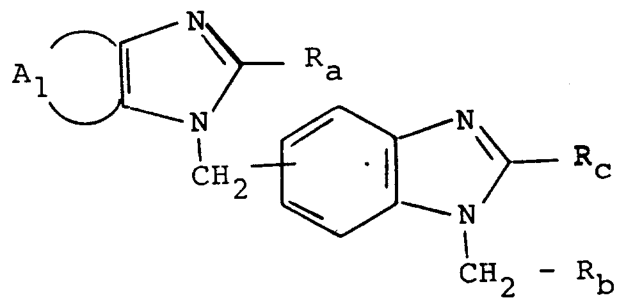

,(VII)

mit einer Verbindung der allgemeinen Formel

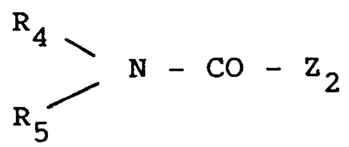

,(VIII)

in denen
$R_a$ bis $R_c$, $R_4$ und $R_5$ wie in den Ansprüchen 1 bis 5 definiert sind,
$A_1$ eine 1,4-Butadienylengruppe, die durch $R_1$ und durch eine $R_3NH$-Gruppe substituiert ist, wobei $R_1$ und $R_3$ wie in den Ansprüchen 1 bis 5 definiert sind, und
$Z_2$ eine nukleophile Austrittsgruppe oder auch
$Z_2$ und $R_5$ zusammen eine Stickstoff-Kohlenstoff-Bindung darstellen, umgesetzt wird oder
f) zur Herstellung einer Verbindung der allgemeinen Formel I, in der $R_2$ eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können, eine Phthalimino- oder Homophthaliminogruppe, wobei eine Carbonylgruppe in einer Phthaliminogruppe durch eine Methylengruppe ersetzt sein kann, eine 5-, 6- oder 7-gliedrige Alkyleniminogruppe, in welcher eine Methylengruppe durch eine Carbonyl- oder Sulfonylgruppe ersetzt ist, eine Glutarsäureiminogruppe, in der die n-Propylengruppe durch ein oder zwei Alkylgruppen mit jeweils 1 bis 3 Kohlenstoffatomen oder durch eine Tetramethylen- oder Pentamethylengruppe substituiert sein kann, oder eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können, darstellt, eine Verbindung der allgemeinen Formel

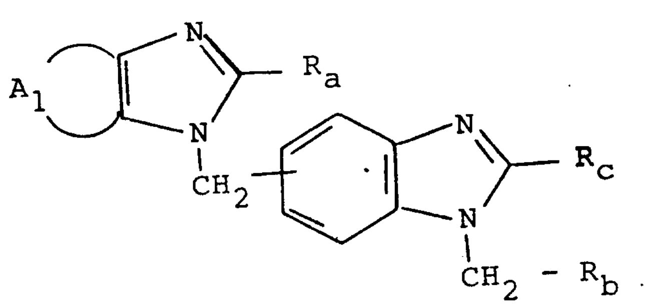

,(VII)

mit einer Verbindung der allgemeinen Formel

$Z_3$ - U - $R_6$ ,(IX)

in denen
$R_a$ bis $R_c$ wie in den Ansprüchen 1 bis 5 definiert sind,
$A_1$ eine 1,4-Butadienylengruppe, die durch $R_1$ und durch eine $R_3NH$-Gruppe substituiert ist, wobei

$R_1$ und $R_3$ wie in den Ansprüchen 1 bis 5 definiert sind,
$Z_3$ eine Hydroxygruppe oder eine nukleophile Austrittsgruppe, U eine Carbonyl- oder Sulfonylgruppe und
$R_6$ eine Alkylgruppe mit 1 bis 4 Kohlenstoffatomen, eine Phenyl-, o-Hydroxycarbonylphenyl-, o-Hydroxycarbonylphenylmethyl- oder o-Hydroxycarbonylmethylphenylgruppe, eine gegebenenfalls durch eine oder zwei Alkylgruppen mit jeweils 1 bis 3 Kohlenstoffatomen oder durch eine Tetramethylen- oder Pentamethylengruppe substituierte 3-Hydroxycarbonylpropylgruppe oder eine gegebenenfalls durch eine Alkylgruppe mit 1 bis 3 Kohlenstoffatomen oder durch eine Phenylgruppe mono- oder disbustituierte 2-Hydroxycarbonylethenylgruppe, in der die Substituenten gleich oder verschieden sein können, oder
$R_3$ und $R_6$ zusammen eine n-Propylen-, n-Butylen- oder n-Pentylengruppe bedeuten, oder, falls $Z_3$ eine Hydroxygruppe darstellt, mit deren reaktionsfähigen Derivaten umgesetzt wird und
erforderlichenfalls ein während der Umsetzungen a) bis f) zum Schutze von reaktiven Gruppen verwendeter Schutzrest abgespalten wird und/oder
gewünschtenfalls anschließend ein so erhaltenes Gemisch von Stellungsisomeren einer Verbindung der allgemeinen Formel I mittels Isomerentrennung aufgetrennt wird oder
eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.